# EUROPEAN PATENT SPECIFICATION

(11) **EP 4 021 441 B1**
(45) Date of publication and mention of the grant of the patent: **14.10.2026**
(21) Application number: 20858588.5
(22) Date of filing: 31.08.2020
(51) Int. Cl.: A61K 31/403, A61K 31/4045, A61K 31/431, A61P 31/04, A61K 45/06, C07D 209/04, C07D 215/233, C07D 215/44, C07D 239/94, C07D 401/12, C07D 417/12, C07D 471/04

(54) **MEMBRANE-ACTIVE ANTI-BACTERIAL COMPOUNDS AND USES THEREOF**
MEMBRANAKTIVE ANTIBAKTERIELLE VERBINDUNGEN UND VERWENDUNGEN DAVON
COMPOSÉS ANTIBACTÉRIENS À MEMBRANE ACTIVE ET LEURS UTILISATIONS

(30) Priority: 30.08.2019 US 201962894135 P
(43) Date of publication of application: 06.07.2022
(73) Proprietor: Trustees of Dartmouth College, Hanover, NH 03755-1404 (US); Regents of the University of Minnesota, Minneapolis, MN 55455-2020 (US)
(72) Inventor: CHEUNG, Ambrose, Hanover, NH 03755-1404 (US); ALDRICH, Courtney, Minneapolis, MN 55455-2020 (US); SCHULTZ, John, Minneapolis, MN 55455-2020 (US)
(74) Representative: Murgitroyd & Company
(86) International application number: PCT/US2020/048776
(87) International publication number: WO 2021/042046

(56) References cited:
- WO-A1-2019/042470
- WO-A2-2011/032050
- YAMASHKIN S A ET AL: "Tautomerism in the series of pyrrolo[2,3-h]-, -[3,2-f]-, -[2,3-f]-, -[3,2-g]-, AND -[3,2-h]quinolines", CHEMISTRY OF HETEROCYCLIC COMPOUNDS, KLUWER ACADEMIC PUBLISHERS-PLENUM PUBLISHERS, NL, vol. 45, no. 9, 24 November 2009 (2009-11-24), pages 1121 - 1130, XP019767872, ISSN: 1573-8353, DOI: 10.1007/S10593-009-0399-Z
- SILVIJA MARACIC ET AL: "Quinoline and ferrocene conjugates: Synthesis, computational study and biological evaluations", APPLIED ORGANOMETALLIC CHEMISTRY, LONGMAN GROUP UK, LTD, HOBOKEN, USA, vol. 33, no. 1, 19 November 2018 (2018-11-19), pages n/a, XP071533646, ISSN: 0268-2605, DOI: 10.1002/AOC.4628
- MARC MARULL ET AL: "Selective and Efficient Structural Elaboration of 2-(Trifluoromethyl)quinolinones", EUROPEAN JOURNAL OF ORGANIC CHEMISTRY, WILEY-VCH, DE, vol. 2003, no. 8, 27 March 2003 (2003-03-27), pages 1576 - 1588, XP072102427, ISSN: 1434-193X, DOI: 10.1002/EJOC.200390217
- PANDA SIVA S ET AL: "New trifluoromethyl quinolone derivatives: Synthesis and investigation of antimicrobial properties", BIOORGANIC & MEDICINAL CHEMISTRY LETTERS, ELSEVIER, AMSTERDAM NL, vol. 23, no. 11, 3 April 2013 (2013-04-03), pages 3225 - 3229, XP028535171, ISSN: 0960-894X, DOI: 10.1016/J.BMCL.2013.03.120
- "Encyclopedia of reagents for organic synthesis; Vol. 7 : Sod - Trim", 1 January 2005, WILEY, Chichester, ISBN: 978-0-470-84289-8, article NARSAIAH B. ET AL: "Ethyl 4,4,4-Trifluoroacetoacetate", XP093111055, DOI: 10.1002/047084289X.rn00587
- MANFRED SCHLOSSER ET AL: "Relay Propagation of Crowding: The Trifluoromethyl Group as Both an Emitter and Transmitter of Steric Pressure", EUROPEAN JOURNAL OF ORGANIC CHEMISTRY, WILEY-VCH, DE, vol. 2006, no. 3, 30 November 2005 (2005-11-30), pages 729 - 734, XP072112119, ISSN: 1434-193X, DOI: 10.1002/EJOC.200500728
- NGUYEN ET AL.: "Preparation and antibacterial evaluation of decarboxylated fluoroquinolones", BIOORGANIC AND MEDICINAL CHEMISTRY LETTERS, vol. 21, 2011, pages 5961 - 5963, XP028286404, DOI: 10.1016/j.bmcl.2011.07.060
- DATABASE PubChem compound 10 June 2016 (2016-06-10), "1(2H)-Isoquinolinone, 8-(methylthio)-", XP055796994, retrieved from NCBI Database accession no. 119085983
- DOMAGALA: "Structure-activity and structure-side-effect relationships for the quinolone antibacterials", JOURNAL OF ANTIMICROBIAL CHEMOTHERAPY, vol. 33, 1994, pages 685 - 706, XP000945006, DOI: 10.1093/jac/33.4.685

## Description

This invention was made with government support under R21 AI130540 awarded by the National Institutes of Health. The government has certain rights in the invention.

### BACKGROUND

The emergence of multidrug-resistant (MDR) gram-positive bacteria is on the rise. Although antibiotic stewardship and infection control measures are helpful, new agents against MDR gram-positive bacteria are needed. Various embodiments of the present disclosure address the aforementioned needs.

Eur. J. Org. Chem. 2003, 1576-1588, Marull et al, discusses 5-methoxy-2-(trifluoromethyl)- 4(1H)-quinolinone or 5-fluoro-2-(trifluoromethyl)- 4(1H)-quinolinone. "Ethyl 4,4,4-Trifluoroacetoacetate" in: "Encyclopedia of reagents for organic synthesis; Vol. 7 : Sod - Trim" Narsaiah et al, references 2-trifluoromethyl-4-quinolone (4) (D6 p.1 col.2), as an antimalarial agent. Eur. J. Org. Chem. 2006, 729-734, Schlosser et al, discusses 5,7-dimethyl-2-(trifluoromethyl)-4(1H)-quinolinone (p.732 col.2 §5).

### SUMMARY

The invention is as defined in the claims.

Any reference in the description to a method of treatment of the human (or animal) body by therapy (or for diagnosis), to the use in a treatment, to a therapy is to be interpreted as reference to compounds, pharmaceutical compositions or combinations of the present invention for use in such a method

### DESCRIPTION OF THE DRAWINGS

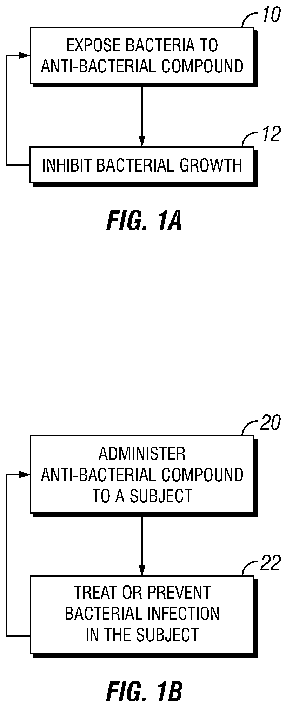

**FIG. 1A** illustrates a method to inhibit bacterial growth according to an aspect of the present disclosure.
**FIG. 1B** illustrates a method of treating or preventing a bacterial infection in a subject by administering to the subject an anti-bacterial compound of the present disclosure.

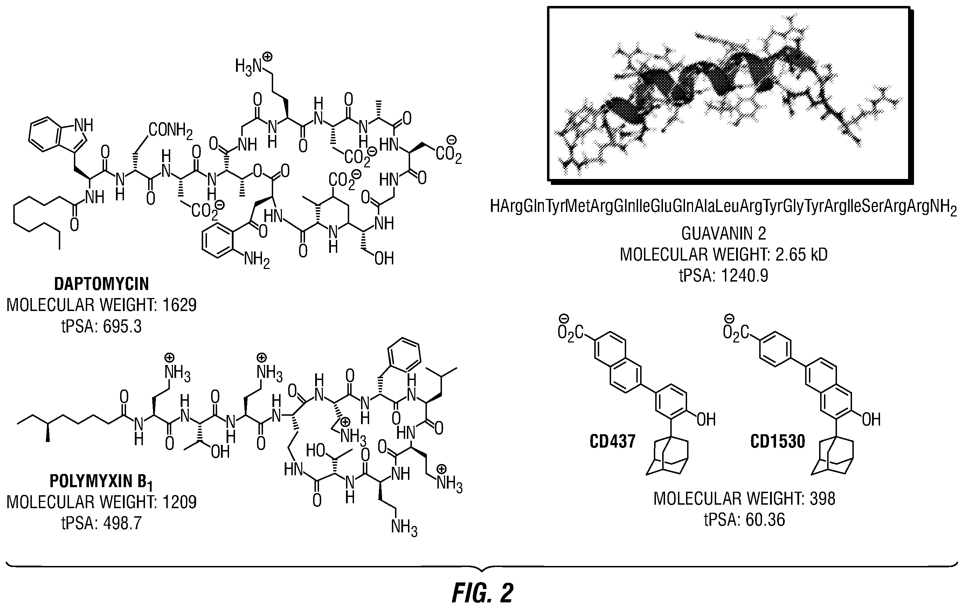

**FIG. 2** illustrates therapeutic and experimental membrane-disrupting agents. Daptomycin is a Food and Drug Administration (FDA)-approved antibiotic for treatment of gram-positive bacteria that inserts into the cytoplasmic membrane of the bacteria and permeabilizes it via membrane-associated oligomers. Guavanin 2 is a cationic antimicrobial peptide (CAMP) that disrupts membranes of bacteria via membrane hyperpolarization. The Guavanin 2 structure is taken from PDB 5V1E. Polymyxin B₁ is part of the polymyxin class of antibiotics and is an FDA-approved antibiotic that disrupts membranes of gram-positive bacteria.

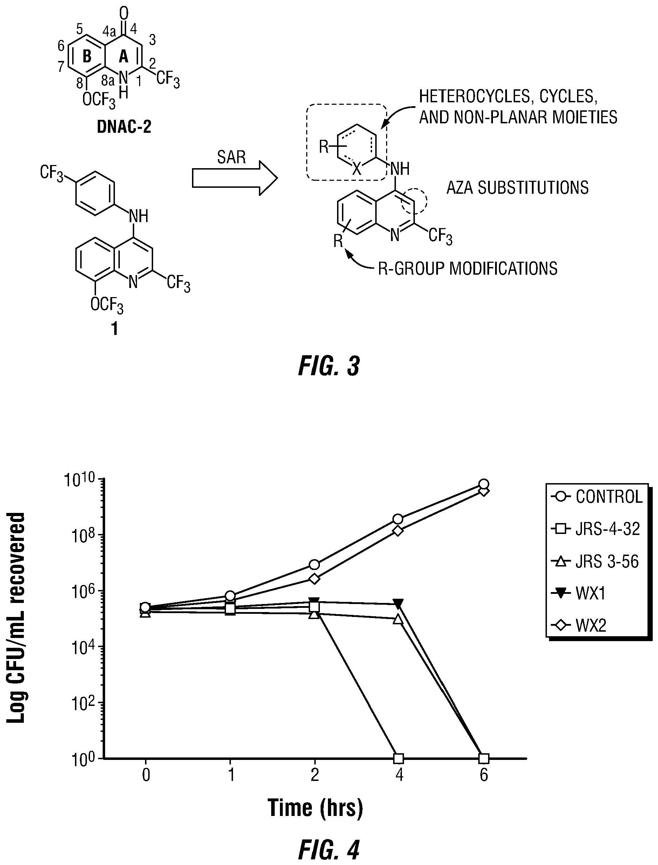

**FIG. 3** illustrates high throughput screen (HTS) hits and structure-activity relationships (SAR) analysis of a 4-aminoquinoline scaffold.
**FIG. 4** illustrates *in vitro* time-kill analysis of methicillin resistant *Staphylococcus aureus* (MRSA). Bacterial killing was monitored by measuring the colony forming unit (CFU) for six hours when treated with compounds **22, 31, 117,** and **123** (compounds **22** and **31** are not of the invention) at 1× the minimum inhibitory concentration (MIC) of each compound. The CFU at each time point was determined by plating and then compared to a dimethyl sulfoxide (DMSO) control.

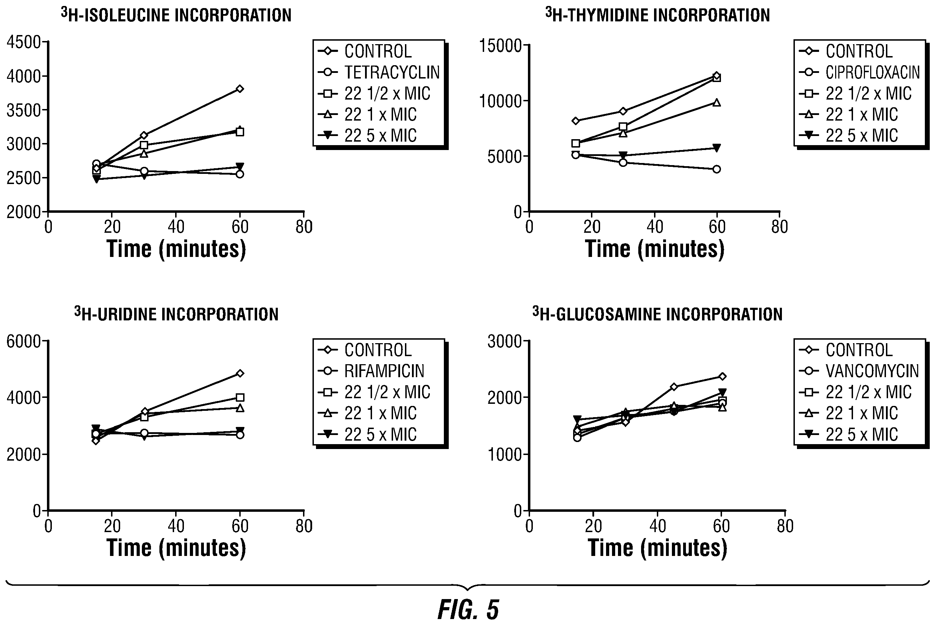

**FIG. 5** illustrates macromolecular synthesis assays (representative data with **123).** Time-course for inhibition of incorporation of radiolabeled precursors [³H]-L-isoleucine (protein), [³H]-thymidine (DNA), [³H]-uridine (RNA), and [³H]-glucosamine (cell wall) in *S*. *simulans* by **123** at 0.5×, 1× and 5× MIC. Data are expressed as the percentage of inhibition relative to the DMSO only negative control. The positive control antibiotics denoted by closed circles were used at 10× MIC. Data represent the mean ± standard deviation (SD) of triplicate experiments.

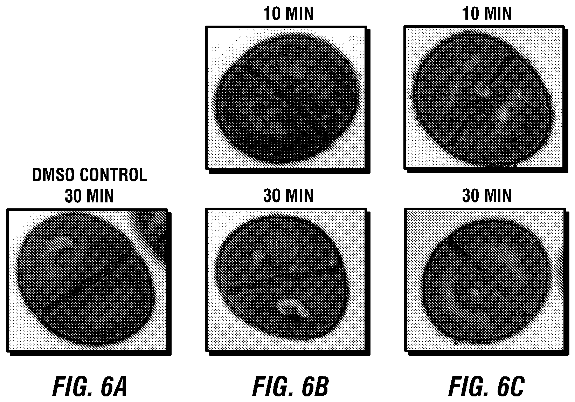

**FIGS. 6A, 6B** and **6C** illustrate transmission electron microscopy (TEM) imaging of MRSA USA300 treated with compounds **22** and **31** (not of the invention). Membrane disruption are highlighted with arrows by compounds **22 (****FIG. 6B****)** and **31 (****FIG. 6C****)** after 10 minutes (top row) and 30 minutes (bottom row) of exposure at 1× MIC compared to a DMSO control **(****FIG. 6A****).**

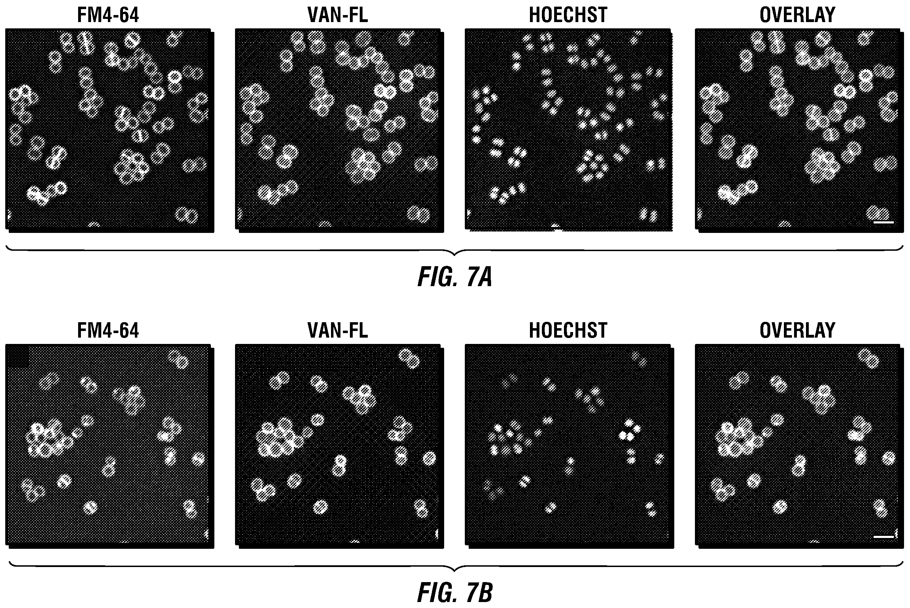

**FIGS. 7A** and **7B** illustrate fluorescent microscopy (FM) analysis of the membrane, cell wall, and DNA in MRSA. FM of COL MRSA strain treated with compound **123 (****FIG. 7B****,** bottom row) at 1× MIC and DMSO **(****FIG. 7A****,** top row) for 30 minutes followed by staining with FM-64 (far left column, 0.5 µg/mL), VanFL (second column, 1 µg/mL), and Hoechst (third column, 1 µg/mL) for 5 minutes and washed with 1× phosphate buffered saline (PBS) before imaging (fourth column, overlay).

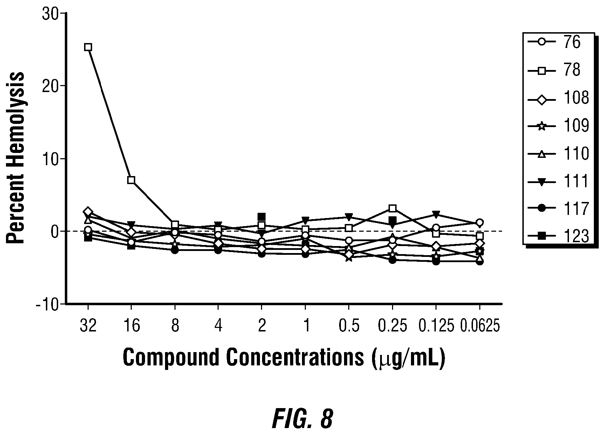

**FIG. 8** illustrates percent hemolysis analysis of sheep erythrocytes. Concentration-dependent hemolysis was measured by monitoring the optical density (OD)₅₄₀ of PBS-washed sheep erythrocytes. Complete hemolysis (100%) was confirmed by treatment of erythrocytes with Triton X-100. Data points represent the mean ± SD of triplicate experiments.

### DETAILED DESCRIPTION

It is to be understood that both the foregoing general description and the following detailed description are illustrative and explanatory, and are not restrictive of the subject matter, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that include more than one unit unless specifically stated otherwise.

The section headings used herein are for organizational purposes and are not to be construed as limiting the subject matter described. In the event that one or more of the literature mentioned herein and similar materials defines a term in a manner that contradicts the definition of that term in this application, this application controls.

Bacterial infections present significant public health concerns. For instance, nosocomial infections caused by resistant Gram-positive (Gram⁺) organisms are on the rise, presumably due to a combination of factors including prolonged hospital exposure, increased use of invasive procedures, and pervasive antibiotic therapy. Compounding the problem is the emergence of multidrug-resistant (MDR) Gram⁺ bacteria, such as methicillin-resistant *Staphylococcus aureus* (MRSA), *Staphylococcus epidermidis, Streptococcus pneumoniae,* and *Enterococcus* spp., which render treatment extremely difficult.

As a result, last resort antibiotics, such as vancomycin, linezolid, and daptomycin are frequently used as a treatment for infections caused by MDR Gram⁺ bacteria, which have the unintended consequence of selecting resistance to these agents. Although antibiotic stewardship and infection control measures are helpful, new anti-bacterial agents against MDR Gram⁺ bacteria are needed.

More generally, a need exists for more effective anti-bacterial compounds and methods for inhibiting the growth of various bacteria and treating various bacterial infections. Embodiments of the present disclosure address the aforementioned need.

The present disclosure pertains to methods of inhibiting bacterial growth. As illustrated in **FIG. 1A****,** the methods of the present disclosure generally include one or more of the following steps of: exposing bacteria to an anti-bacterial compound (step 10); and inhibiting bacterial growth (step 12). As illustrated in **FIG. 1B****,** the exposing can occur via administration of the anti-bacterial compound to a subject (step 20) in order to treat or prevent a bacterial infection in a subject (step 22).

The methods of the present disclosure can be repeated until the bacteria and/or the bacterial infection are eliminated. The anti-bacterial compound exposed to the bacteria has the following base structure (only those compounds as defined in the claims being part of the invention):

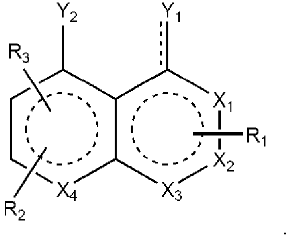

As set forth in more detail herein, the methods and anti-bacterial compounds of the present disclosure can have numerous variations. For instance, the methods of the present disclosure can utilize various anti-bacterial compounds that have various chemical structures and functional groups. Furthermore, the anti-bacterial compounds of the present disclosure may be utilized to inhibit bacterial growth in various subjects for the treatment or prevention of various bacterial infections in the subject.

### Anti-Bacterial Compounds

The methods of the present disclosure can utilize various types of anti-bacterial compounds for inhibition of bacterial growth. Moreover, the anti-bacterial compounds of the present disclosure can include various chemical configurations and functional groups.

For example, the anti-bacterial compounds of the present disclosure have the following general structure (only those compounds as defined in the claims being part of the invention):

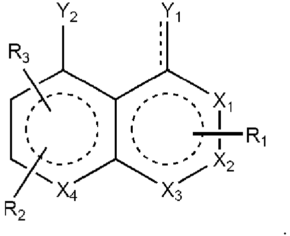

Y₁ includes, without limitation, O, OH, H, F, R₄-O, R₄-NMe, R₄-N(C=O), R₄-NH, CF₃, CHF₂, CH₂F, alky(C₁-C₄), methoxy, thiomethyl, cyano, nitro, fluoro, chloro, bromo, iodo, cycloalkyl(C₃-C₆), a heterocyclic, an aromatic, a morpholine, a pyrrolidine, an indolone, an alkyl, a cycloalkyl, an alkenyl, a cycloalkenyl, an alkynyl, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, a phenyl, a pyridyl, an amino, a pyridyl amino, a pyridazine, a ketone, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, piperazinyl, 3,4-dichloroanilino,3,4-difluoroanilino, 5-(trifluoromethyl) pyridin-2-yl-amino, piperazin-1-yl, 5-(trifluoromethyl)pyridin-2-yl-amino, thiazol-2-ylamino, 5-(trifluoromethyl)pyridin-2-yl-amino, 3,4-dichloroanilino, 3-(trifluoromethoxy)aniline, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1H-indazol-4-yl-amino, 4-(trifluoromethyl)cyclohexyl, 4-(trifluoromethyl)cyclohexyl-amino, 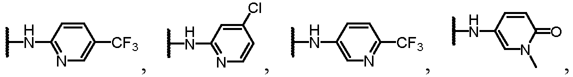 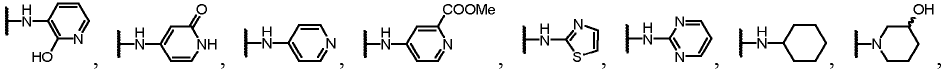 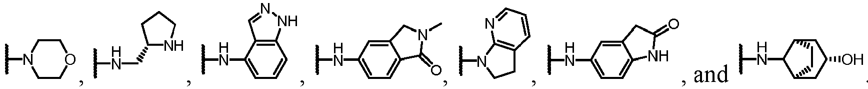 In the compounds of the invention, Y₁ is O.

Y₂ includes, without limitation, H, F, R₄-O, R₄-NMe, R₄-N(C=O), R₄-NH, CF₃, Cl, Br, cyano, nitro, CF₃, CHF₂, CH₂F, a heterocyclic, an aromatic, a morpholine, a pyrrolidine, an indolone, an alkyl, a cycloalkyl, an alkenyl, a cycloalkenyl, an alkynyl, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, a phenyl, a pyridyl, an amino, a pyridyl amino, a pyridazine, a ketone, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, piperazinyl, 3,4-dichloroanilino, 3,4-difluoroanilino, 3-(trifluoromethoxy)aniline, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H-*indazol-4-yl-amino, 4-(trifluoromethyl)cyclohexyl-amino, pyrrol-2-yl, pyrrol-3-yl, pyrrol-2-ylamino, 1-methylpyrrol-2-yl, 1-methylpyrrol-3-yl, 1-methylpyrrol-2-ylamino, piperazinyl morpholino, R₆-NH, alky(C₁-C₄), cycloalkyl(C₃-C₆), methoxy, thiomethyl, 3-(trifluoromethoxy)anilino, 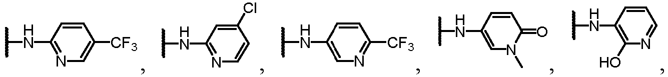 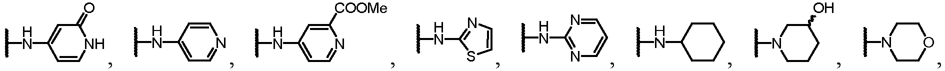 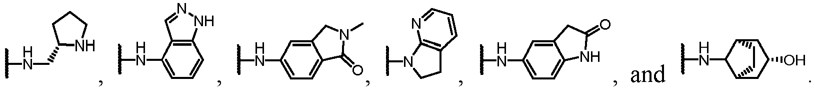 R₆ can be 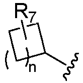 and n can be an integer that can be 0 to 5. R₇ can include, without limitation, H, F, CF₃, CHF₂, CH₂F, alky(C₁-C₄), methoxy, thiomethyl, and cyano. Y₂ can include, without limitation, an aryl thiol, a disulfide, a sulfoxide, a sulfone, a sulfonamide, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, piperazinyl, piperazin-1-yl, and combinations thereof. In the compounds of the invention, Y₂ is as defined in the claims.

X₁, X₂, X₃, and X₄ each independently include, without limitation, C, CH, CF, C-R₉, N, NH, and N-R₉. In the compounds of the invention, X₁, X₂, X₃, and X₄ are as defined in the claims.

R₁, R₂, R₃, R₄, and R₉, can each independently include, without limitation, H, F, -CHF₂, -CH₂F, -CF₃, -OCF₃, -OMe, -S(O)₂N(Me)₂, azido, cyano, nitro, thiomethyl, methoxy, alkyl(C₁-C₄)ester, alky(C₁-C₄), chloro, bromo, fluoro, iodo ethyl, benzylether, methoxy, a benzonitrile, a pyridyl, a pyridyl amino, an aniline, an amino, piperazinyl, a morpholine, a pyrrolidine, an indolone, an anilino, 3,4-difluorophenyl, a pyridazine, a heterocyclic, an aromatic, an alkyl, a cycloalkyl, an alkenyl, a cycloalkenyl, an alkynyl, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, phenyl, a ketone, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, thiomethyl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, thiazol-2-yl, 4-(trifluoromethyl)cyclohexyl, 3,4-dichlorophenyl, 4-trifluoromethylphenyl, 5-(trifluoromethyl)pyridin-2-yl-amino, 3,4-dichloroanilino, 3,4-difluoroanilino, 3-(trifluoromethoxy)aniline, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1H-indazol-4-yl-amino, 4-(trifluoromethyl)cyclohexyl-amino, nitro, cyano, carboxyl, C(O)OMe, C(O)OEt, azido, R₆-NH, and morpholino. R₆ can be 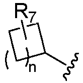 and n can be an integer that can be between 0 to 5. R₇ can include, without limitation, H, F, CF₃, CHF₂, CH₂F, alky(C₁-C₄), methoxy, thiomethyl, and cyano. R₁, R₂, R₃, R₄, and R₉, can include, without limitation, -C(=O)OH, tetrazolyl, 5-(fluoro)pyridin-2-yl, 5-dimethylaminopyridin-2-yl, 6-(trifluoromethyl)pyridazine-3-yl, 6-(fluoro)pyridazine-3-yl, (N,N-dimethyl-6-sulfamoyl)pyridin-2-yl, 1*H*-indazol-4-yl, 4-(trifluoromethyl)cyclohexyl, thiazol-2-yl, tetrazolyl, azido, cyano, nitro, thiomethyl, methoxy, alkyl(C₁-C₄)ester, alky(C₁-C₄), thiazol-2-yl-amino, and combinations thereof. In the compounds of the invention, R₁, R₂, R₃ and R₄ are as defined in the claims.

The anti-bacterial compounds of the present disclosure include, without limitation: 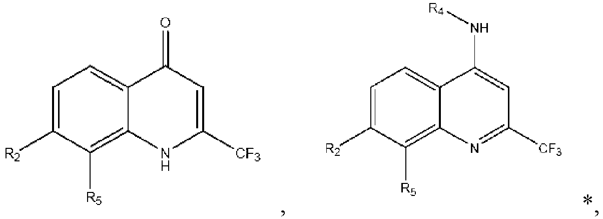 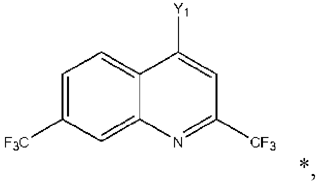 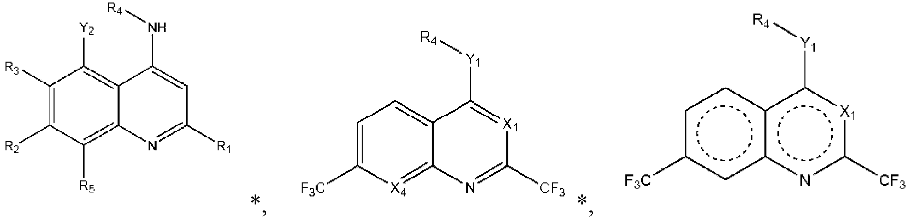 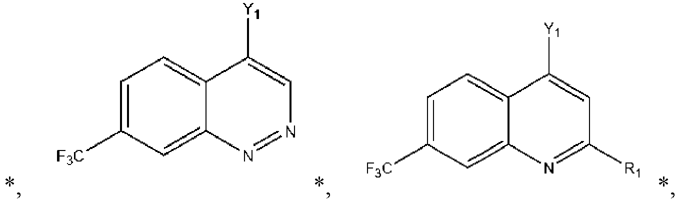 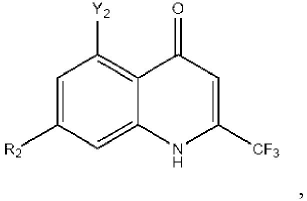 tautomers thereof, and combinations thereof (* = not of the invention; the remaining compounds are of the invention when their substituents are as defined in the claims).

The anti-bacterial compounds of the present disclosure include the following structure (which is only of the invention when the substituents are as defined in the claims):

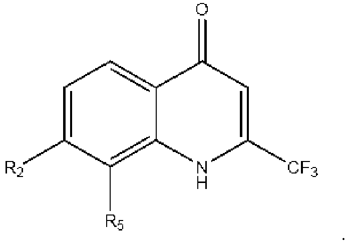

R₂ and R₅ can each independently include, without limitation, H, F, CF₃, OCF₃, chloro, bromo, ethyl, benzylether, methoxy, thiomethyl, nitro, cyano, carboxyl, C(O)OMe, C(O)OEt, and azido. The anti-bacterial compound can include a tautomer of the aforementioned structure.

The anti-bacterial compounds of the present disclosure include the following structure (which is not of the invention):

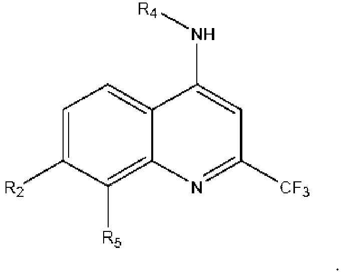

R₂, R₄, and R₅ can each independently include, without limitation, H, CF₃, OCF₃, OMe, S(O)₂N(Me)₂, 3-(trifluoromethoxy)phenyl, phenyl, 2-isopropylphenyl, 3-acetylphenyl, 3-benzonitrile, 2-chlorophenyl, 3-chlorophenyl, 4-chlorophenyl, 3-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, and 3,4-dichlorophenyl. R₂ and R₅ can include, without limitation, H, CF₃, OCF₃, OMe, S(O)₂N(Me)₂. R₄ can include, without limitation, H, Me, phenyl, cyclohexyl, pyridin-2-yl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 4-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, 1*H*-indazol-4-yl, 4-(trifluoromethyl)cyclohexyl, and thiazol-2-yl. The anti-bacterial compound can include a tautomer of the aforementioned structure.

The anti-bacterial compounds of the present disclosure include the following structure (which is not of the invention):

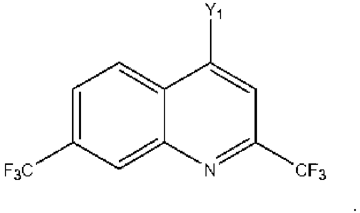

Y₁ can include, without limitation: 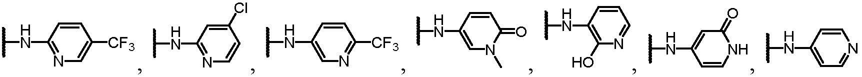 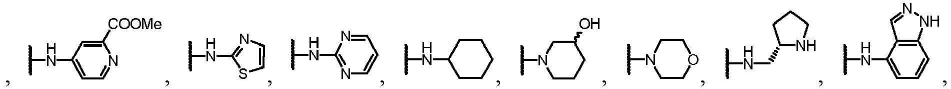 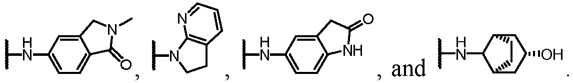 Y₁ can include, without limitation, thiazol-2-ylamino, 5-(trifluoromethyl)pyridin-2-yl-amino, 3,4-dichloroanilino, 3,4-difluoroanilino, 3-(trifluoromethoxy)aniline, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl, and 4-(trifluoromethyl)cyclohexyl-amino. The anti-bacterial compound can include a tautomer of the aforementioned structure.

The anti-bacterial compounds of the present disclosure include the following structure (which is not of the invention):

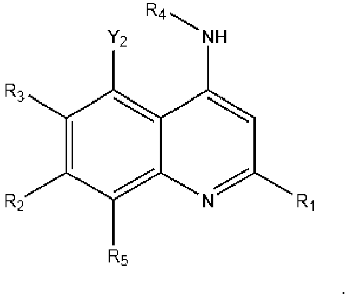

Y₂ can include, without limitation, H and F.

R₁, R₂, R₃, R₄, and R₅ can each independently include, without limitation, H, F, Cl, Br, cyano, nitro, CF₃, CHF₂, 3,4-dichlorophenyl, 3-(trifluoromethoxy)phenyl, and 5-(trifluoromethyl)pyridin-2-yl. R₄ can include, without limitation, 3,4-difluorophenyl, 3,4-dichlorophenyl, 3-(trifluoromethoxy)phenyl, and 5-(trifluoromethyl)pyridin-2-yl. The anti-bacterial compound can include a tautomer of the aforementioned structure.

The anti-bacterial compounds of the present disclosure include the following structure (which is not of the invention):

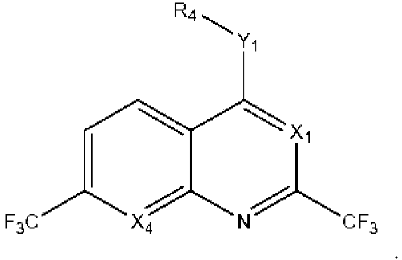

Y₁ can include, without limitation, O, NMe, N(C=O), and NH.

X₁ and X₄ can each independently include, without limitation, CH and N.

R₄ can include, without limitation, 3-chlorophenyl, 3-fluorophenyl, 3,4-dichlorophenyl, 3-(trifluoromethoxy)phenyl, 4-trifluoromethylphenyl, 5-(trifluoromethyl)pyridin-2-yl, and 3,4-difluorophenyl. The anti-bacterial compound can include a tautomer of the aforementioned structure.

The anti-bacterial compounds of the present disclosure include the following structure (which is not of the invention):

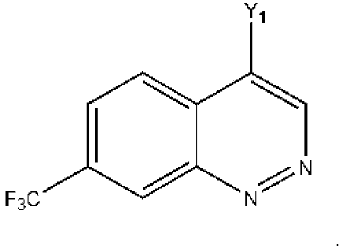

Y₁ can include, without limitation, 3,4-difluoroanilino and 5-(trifluoromethyl) pyridin-2-yl-amino.

The anti-bacterial compounds of the present disclosure include the following structure (which is not of the invention):

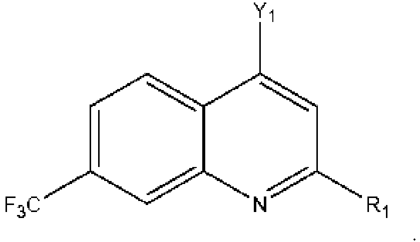

Y₁ can include, without limitation, H, F, Cl, Br, CF₃, CHF₂, CH₂F, piperazinyl, 5-(trifluoromethyl)pyridin-2-yl-amino, and morpholino.

R₁ can include, without limitation, H, F, Cl, Br, CF₃, CHF₂, CH₂F, piperazinyl, 5-(trifluoromethyl)pyridin-2-yl-amino, and morpholino. The anti-bacterial compound can include a tautomer of the aforementioned structure.

The anti-bacterial compounds of the present disclosure include the following structure (which is of the invention when the substituents are as defined in the claims):

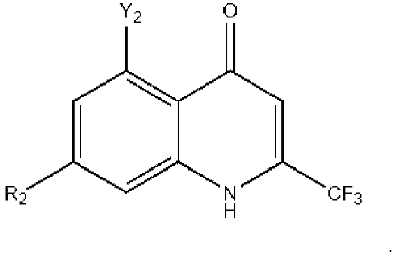

Y₂ can include, without limitation, H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, alky(C₁-C₄), phenyl, cyclohexyl, pyridin-2-yl, 3,4-dichloroanilino, 3,4-difluoroanilino, 3-(trifluoromethoxy)aniline, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, pyridazine-3-yl-amino, pyridazine-3-yl, (N,N-dimethyl-6-sulfamoyl)pyridin-2-yl-amino, 1*H*-indazol-4-yl-amino, 4-(trifluoromethyl)cyclohexyl-amino, pyrrol-2-yl, pyrrol-3-yl, pyrrol-2-ylamino, 1-methylpyrrol-2-yl, 1-methylpyrrol-3-yl, 1-methylpyrrol-2-ylamino, piperazinyl, and morpholino.

R₂ can include, without limitation, F, CF₃, 3,4-dichloroanilino, 3,4-difluoroanilino, 3-(trifluoromethoxy)aniline, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl-amino, and morpholino. The anti-bacterial compound can include a tautomer of the aforementioned structure.

The anti-bacterial compounds of the present disclosure include the following structure (only those compounds as defined in the claims being part of the invention):

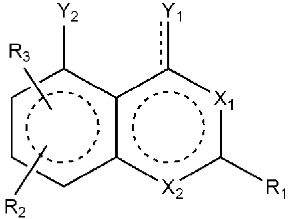

Y₁ and Y₂ can be any of the above mentioned Y₁ and Y₂ functional groups. X₁ and X₂ can be any of the X₁, X₂, X₃, or X₄ groups as discussed above. R₁, R₂, and R₃, can be any of the aforementioned R₁, R₂, R₃, R₄, and R₅ groups.

Y₁ can include, without limitation, =O, -OH, -H, -F, -O-R₄, -N(Me)-R₄, -N(C=O)R₄, - C(=O)R₄, -NH-R₄, -CF₃, -CHF₂, -CH₂F, alky(C₁-C₄), methoxy, thiomethyl, cyano, nitro, fluoro, chloro, bromo, iodo, cycloalkyl(C₃-C₆), an alkenyl(C₃-C₆) group, a cycloalkenyl(C₃-C₆) group, an alkynyl(C₃-C₆) group, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, a phenyl, a pyridyl, an amino, a pyridyl amino, an indolone, a pyridazine, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, and piperazinyl, 3,4-dichloroanilino, 3,4-difluoroanilino, 5-(trifluoromethyl)pyridin-2-yl-amino, piperazin-1-yl, 5-(trifluoromethyl)pyridin-2-yl-amino, morpholino, 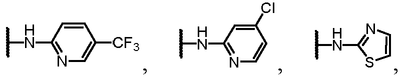 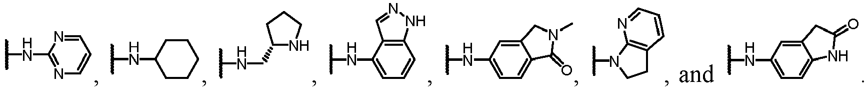 In the compounds of the invention, Y₁ is O.

Y₂ can include, without limitation, -S-R₄, -O-R₄, -O-N=CH-R₄, -N(Me)-R₄, - N(C=O)R₄, -NH-R₄, -C(=O)R₄, -C(=O)O-R₄, -NH-R₄-C(=O)OH, -NH-R₄-C(=O)NH₂, -NH-R₄-NO₂, -NH-R₄-CN, -NH-R₄-CF₃, -NH-R₄-F, -NH-R₄-CHF₂, -NH-R₄-CH₂F, -R₄-alky(C₁-C₄), -H, -F, -CF₃, -CHF₂, -CH₂F, alky(C₁-C₄), a heterocyclic, an aromatic, methoxy, thiomethyl, cyano, nitro, chloro, bromo, iodo, cycloalkyl(C₃-C₆), an alkenyl(C₃-C₆) group, a cycloalkenyl(C₃-C₆) group, an alkynyl(C₃-C₆) group, a cycloalkyl(C₃-C₇)oxy, an alkyl(C₁-C₄)oxycarbonyl, alkyl(C₁-C₄), an alkyl(C₁-C₄)oxycarbonyloxymethyl group, a branched alkyl(C₄-C₈)oxycarbonyloxymethyl group, phenyl, an aryl alcohol, a phenylalkyl(C₁-C₄), an aryl halo, a pyridyl, a pyridyl amino, an indolone, a pyridazine, an aryl ketone, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, an aryl thiol, a disulfide, a sulfoxide, a sulfone, a sulfonamide, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, and piperazinyl, piperazin-1-yl, morpholino, a thiol, an aryl thiol, a disulfide, a sulfoxide, a sulfone, a sulfonamide, piperazinyl, 3,4-dichloroanilino, 3,4-difluoroanilino, 3-(trifluoromethoxy)aniline, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl-amino, 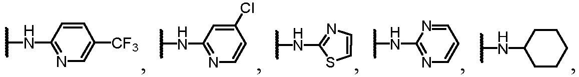 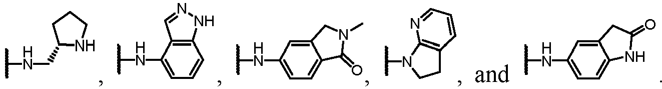 In the compounds of the invention, Y₂ is as defined in the claims.

X₁ and X₂, can include, without limitation, C, CH, CF, C-R₉, N, NH, and N-R₉. In the compounds of the invention, X₁ and X₂, are as defined in the claims.

R₁ can include, without limitation, H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, and alky(C₁-C₄). In the compounds of the invention, R₁ is as defined in the claims.

R₄ and R₉ can include, without limitation, phenyl, pyridin-2-yl, pyridizin-3-yl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 4-fluorophenyl, a bromophenyl group, an iodophenyl group, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, 3,4-dichlorophenyl, 4-trifluoromethylphenyl, 5-(trifluoromethyl)pyridin-2-yl, 5-(fluoro)pyridin-2-yl, 5-dimethylaminopyridin-2-yl, 6-(trifluoromethyl)pyridazine-3-yl, 6-(fluoro)pyridazine-3-yl, (N,N-dimethyl-6-sulfamoyl)pyridin-2-yl, 1*H*-indazol-4-yl, 4-(trifluoromethyl)cyclohexyl, and thiazol-2-yl. In the compounds of the invention, R₄ and R₉ are as defined in the claims.

R₂ and R₃ can include, without limitation, H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, alky(C₁-C₄), chloro, ethyl, benzylether, methoxy, thiomethyl, a benzonitrile, a pyridyl, a pyridyl amino, an aniline, an amino, piperazinyl, a pyrrolidine, an indolone, an anilino, a pyridazine, a heterocyclic, an aromatic, an alkyl, a cycloalkyl, an alkenyl, a cycloalkenyl, an alkynyl, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, phenyl, a ketone, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, thiomethyl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, 3,4-dichlorophenyl, 4-trifluoromethylphenyl, 5-(trifluoromethyl)pyridin-2-yl-amino, 3,4-dichloroanilino, 3,4-difluoroanilino, 3-(trifluoromethoxy)aniline, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl, 4-(trifluoromethyl)cyclohexyl-amino, thiazol-2-yl-amino, and morpholino. In the compounds of the invention, R₂ and R₃ are as defined in the claims.

The anti-bacterial compounds of the present disclosure include the following structure (which is not of the invention):

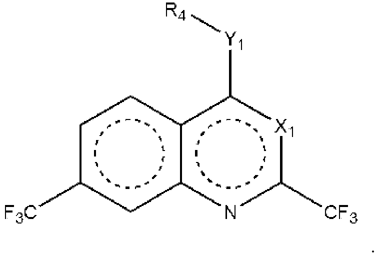

Y₁ can include, without limitation, H, O, NMe, N(C=O), and NH. X₁ can include, without limitation, CH and N. R₄ can include, without limitation, H, Me, phenyl, cyclohexyl, pyridin-2-yl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 4-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3,4-dichlorophenyl, 3-(trifluoromethoxy)phenyl, 4-trifluoromethylphenyl, 5-(trifluoromethyl)pyridin-2-yl, 3,4-difluorophenyl, 1*H*-indazol-4-yl, 4-(trifluoromethyl)cyclohexyl, and thiazol-2-yl.

The anti-bacterial compounds of the present disclosure include the following structure (which is of the invention when the substituents are as defined in the claims):

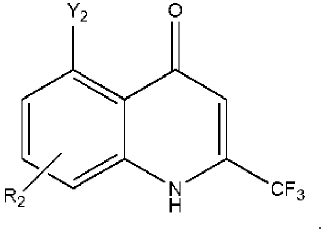

Y₂ and R₂ can each independently include, without limitation, R₆-NH, F, CF₃, CHF₂, CH₂F, alky(C₁-C₄), cycloalkyl(C₃-C₆), methoxy, thiomethyl, cyano, 3,4-dichloroanilino, 3,4-difluoroanilino, 3-(trifluoromethoxy)anilino, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H-*indazol-4-yl-amino, 4-(trifluoromethyl)cyclohexyl-amino, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, and piperazinyl. R₆ can be 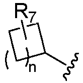 and n is an integer that can be between 0 to 5. R₇ can include, without limitation, H, F, CF₃, CHF₂, CH₂F, alkyl(C₁-C₄), methoxy, thiomehyl, and cuano. The anti-bacterial compound can include a tautomer of the aforementioned structure.

The anti-bacterial compounds of the present disclosure include the following structure (which is of the invention when the substituents are as defined in the claims):

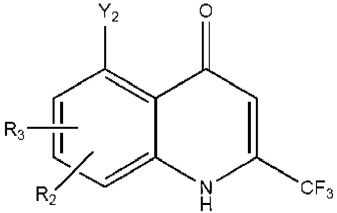

Y₂ and R₂ can each include, without limitation, R₆-NH, H, F, -CHF₂, -CH₂F, -CF₃, - OCF₃, -OMe, -S(O)₂N(Me)₂, -C(=O)OH, tetrazolyl, azido, cyano, nitro, thiomethyl, methoxy, alkyl(C₁-C₄)ester, alky(C₁-C₄),3,4-dichloroanilino, 3,4-difluoroanilino, 3-(trifluoromethoxy)anilino, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl-amino, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, and piperazinyl.

R₃ can include, without limitation, H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, and alky(C₁-C₄). R₆ can be 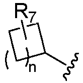 and n is an integer between 0 and 5. R₇ can include, without limitation H, F, Cl, -CHF₂, -CH₂F, -CF₃, -OCF₃, -OMe, -S(O)₂N(Me)₂, - C(=O)OH, tetrazolyl, azido, cyano, nitro, thiomethyl, methoxy, alkyl(C₁-C₄)ester, and alky(C₁-C₄). The anti-bacterial compound can include a tautomer of the aforementioned structure.

The anti-bacterial compounds of the present disclosure include the following structure (which is of the invention when the substituents are as defined in the claims):

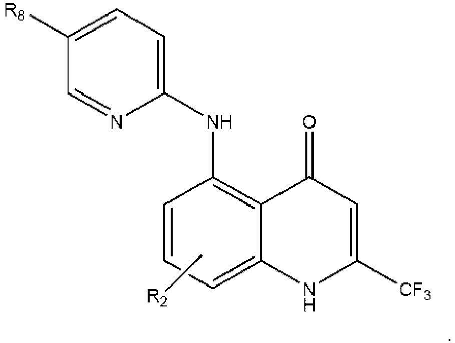

R₈ can include, without limitation, H, F, CF₃, CHF₂, CH₂F, alky(C₁-C₄), methoxy, thiomethyl, cyano, nitro, fluoro, chloro, bromo, and iodo. R₂ can include, without limitation, H, F, OH, CF₃, CHF₂, CH₂F, alky(C₁-C₄), methoxy, thiomethyl, cyano, nitro, fluoro, chloro, bromo, iodo, cycloalkyl(C₃-C₆), morpholino, and piperazinyl. The anti-bacterial compound can include a tautomer of the aforementioned structure.

The anti-bacterial compounds of the present disclosure include the following structure (which is of the invention when the substituents are as defined in the claims):

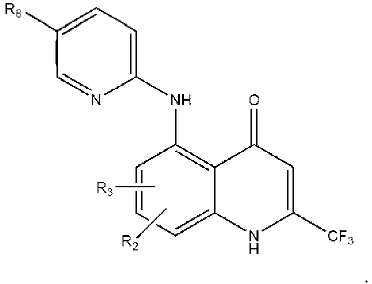

R₂, R₃, and R₈ can include, without limitation, H, F, -CHF₂, -CH₂F, -CF₃, -OCF₃, -OMe, -S(O)₂N(Me)₂, -C(=O)OH, tetrazolyl, azido, cyano, nitro, thiomethyl, methoxy, alkyl(C₁-C₄)ester, alky(C₁-C₄), chloro, bromo, and iodo. The anti-bacterial compound can include a tautomer of the aforementioned structure.

The anti-bacterial compounds of the present disclosure include the following structure (which is of the invention when the substituents are as defined in the claims):

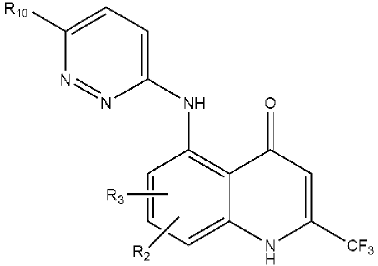

R₁₀ and R₂ can include, without limitation, H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, alky(C₁-C₄), chloro, bromo, iodo, morpholino, and piperazinyl. R₃ can include, without limitation, H, F, -CHF₂, -CH₂F, -CF₃, -OCF₃, -OMe, -S(O)₂N(Me)₂, -C(=O)OH, tetrazolyl, azido, cyano, nitro, thiomethyl, methoxy, alkyl(C₁-C₄)ester, and alky(C₁-C₄). The anti-bacterial compound can include a tautomer of the aforementioned structure.

The anti-bacterial compounds of the present disclosure include the following structure (which is not of the invention):

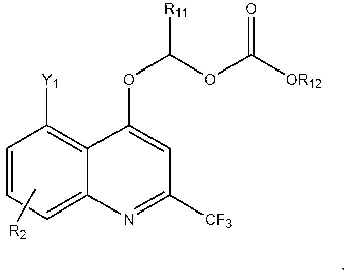

Y₁ and R₂ can include, without limitation, R₆-NH, F, CF₃, CHF₂, CH₂F, alky(C₁-C₄), cycloalkyl(C₃-C₆), methoxy, thiomethyl, cyano, 3,4-dichloroanilino, 3,4-difluoroanilino, 3-(trifluoromethoxy)anilino, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl-amino, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, and piperazinyl. R₁₁ can include, without limitation, H, Me, and alky(C₁-C₄). R₁₂ can include, without limitation, Me, alky(C₁-C₄), cycloalkyl(C₃-C₆), and branched alkyl(C₃-C₆). R₆ can be 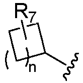 and n can be an integer between 0 to 5. R₇ can include, without limitation, H, F, CF₃, CHF₂, CH₂F, alky(C₁-C₄), methoxy, thiomethyl, and cyano. The anti-bacterial compound can include a tautomer of the aforementioned structure.

In some embodiments, the anti-bacterial compounds of the present disclosure are in a composition. In some embodiments, the compositions of the present disclosure represent therapeutic formulations that enhance or maintain the therapeutic efficacy of the anti-bacterial compounds of the present disclosure.

In some embodiments, the compositions of the present disclosure include one or more stabilizers. In some embodiments, the stabilizers include, without limitation, anti-oxidants, sequestrants, ultraviolet stabilizers, or combinations thereof.

In some embodiments, the compositions of the present disclosure include one or more surfactants. In some embodiments, the surfactants include, without limitation, anionic surfactants, cationic surfactants, zwitterionic surfactants, non-ionic surfactants, or combinations thereof.

In some embodiments, the composition of the present disclosure include one or more excipients. In some embodiments, the excipients include, without limitation, lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, trehalose, sodium alginate, polyvinylpyrrolidone, polyvinyl alcohol, or combinations thereof.

In some embodiments, the compositions of the present disclosure include a delivery vehicle, such as a particle. In some embodiments, the particle includes, without limitation, lipid-based particles, carbon-based particles, metal-based particles, or combinations thereof.

### Inhibition of Bacterial Growth

As set forth in further detail herein, the methods and anti-bacterial compounds of the present disclosure may be utilized to inhibit bacterial growth in various manners via various mechanisms. For instance, in some embodiments, the inhibition of bacterial growth occurs by killing the bacteria. In some embodiments, the inhibition of bacterial growth occurs by disruption of bacterial cell membranes. In some embodiments, the inhibition of bacterial growth occurs by rupturing bacterial cell membranes. In some embodiments, the inhibition of bacterial growth occurs by slowing down bacterial proliferation.

Additionally, as set forth in further detail herein, the methods and anti-bacterial compounds of the present disclosure may be utilized to inhibit the growth of numerous types of bacteria. For instance, in some embodiments, the bacteria includes, without limitation, Gram-positive (Gram⁺) bacteria, antibiotic resistant Gram⁺ bacteria, and combinations thereof. In some embodiments, the bacteria includes, without limitation, *Enterococcus faecium, Staphylococcus epidermidis,* methicillin-resistant *Staphylococcus aureus* (MRSA), methicillin-susceptible *Staphylococcus aureus,* and combinations thereof.

### Exposure of Bacteria to Anti-Bacterial Compounds

The methods of the present disclosure may be utilized to expose the anti-bacterial compounds of the present disclosure to various bacteria in a variety of manners. For instance, the exposing may occur *in vitro.* Alternatively, the exposing may occur *in vivo* in a subject. In some embodiments, the subject is suffering from a bacterial infection.

### Treatment or Prevention of Bacterial Infections

The exposure of bacteria to anti-bacterial compounds can occur by the administration of the anti-bacterial compound to a subject. As such, the present disclosure pertains to methods of treating or preventing a bacterial infection in a subject by administering an anti-bacterial compound of the present disclosure to the subject.

Various methods may be utilized to administer the anti-bacterial compounds of the present disclosure to a subject. For instance, the administering can include, without limitation, intravenous administration, subcutaneous administration, transdermal administration, topical administration, intraarterial administration, intrathecal administration, intracranial administration, intraperitoneal administration, intraspinal administration, intranasal administration, intraocular administration, oral administration, intratumor administration, and combinations thereof.

The anti-bacterial compounds of the present disclosure may be co-administered to a subject with one or more active agents. For instance, the one or more active agents can include oxacillin.

The methods of the present disclosure can be utilized to treat or prevent bacterial infections in various subjects. For instance, the subject may be suffering from a bacterial infection, and the methods of the present disclosure may be utilized to treat the bacterial infection in the subject. Alternatively, the subject may be vulnerable to a bacterial infection, and the methods of the present disclosure may be utilized to prevent the bacterial infection in the subject.

In some embodiments, the subject is a human being. In some embodiments, the subject is an animal, such as cattle, dogs, cats, sheep, cattle, horses, and various livestock.

### Applications and Advantages

The anti-bacterial compounds and methods of the present disclosure can have various advantageous properties and applications. For instance, in some embodiments, the anti-bacterial compounds of the present disclosure possess broad-spectrum anti-Gram⁺ activity. In some embodiments, the anti-bacterial compounds of the present disclosure inhibit the growth and proliferation of bacteria, for example, but not limited to, Gram⁺ bacteria and antibiotic resistant Gram⁺ bacteria.

In some embodiments, the anti-bacterial compounds of the present disclosure have potent antibacterial activity. In some embodiments, the anti-bacterial compounds of the present disclosure enhance the activity of various active agents, such as, for example, oxacillin. In some embodiments, the anti-bacterial compounds of the present disclosure provide for synergy with oxacillin or other active agents.

In some embodiments, the anti-bacterial compounds of the present disclosure can have one or more of the following advantages: (i) the anti-bacterial compounds of the present disclosure provide good solubility; (ii) the anti-bacterial compounds of the present disclosure have intrinsic anti-bacterial activity toward MRSA or other bacterial strains; (iii) the anti-bacterial compounds of the present disclosure provide for sensitizing MRSA to second-generation penicillin; (iv) the anti-bacterial compounds of the present disclosure can have no more than a 4-fold shift in activity upon addition of serum; (v) the anti-bacterial compounds of the present disclosure exhibit no cytotoxicity with towards numerous eukaryotic cells (e.g., Vero cells and other proxy cell systems including, but not limited to, red blood cells); and (vi) the anti-bacterial compounds of the present disclosure exhibit optimal microsomal stability.

Additionally, the anti-bacterial compounds of the present disclosure are rapidly bactericidal, do not select for resistance, and selectively disrupt bacterial membranes over eukaryotic membranes. Furthermore, the anti-bacterial compounds of the present disclosure are non-toxic and display high therapeutic indexes, are devoid of hemolytic activity, and have attractive physicochemical properties that demonstrate anti-bacterial scaffolding for the treatment of Gram⁺ bacterial infections and antibiotic resistant Gram⁺ bacterial infections.

### Additional Embodiments

Reference will now be made to more specific embodiments of the present disclosure and experimental results that provide support for such embodiments. However, Applicants note that the disclosure below is for illustrative purposes only and is not intended to limit the scope of the claimed subject matter in any way.

### Example 1. Development and Characterization of Potent Membrane Disrupting Agents to Combat Antibacterial Resistant Gram-Positive Bacteria

This Example describes the development and characterization of potent membrane disrupting agents to combat antibacterial resistant gram-positive bacteria. In particular, Applicants describe in this Example efforts leading to the identification of 5-aminoquinolone 123 with exceptionally potent gram-positive activity with minimum inhibitory concentrations (MICs) (i.e., ≤ 0.06 µg/mL) against numerous clinical methicillin-resistant *Staphylococcus aureus* (MRSA) isolates.

Preliminary mechanism of action and resistance studies demonstrate that the 5-aminoquinolones are rapidly bactericidal, do not select for resistance, and selectively disrupt bacterial membranes over eukaryotic membranes. The lead compound is non-toxic, displaying a therapeutic index of greater than 1000, is devoid of hemolytic activity, and has attractive physicochemical properties (cLogP = 1.7, MW = 391) that warrant further investigation of this promising antibacterial scaffold for treatment of gram-positive infections.

### Example 1.1. Introduction

Rising antimicrobial resistance (AMR) threatens global public health, and if unabated, a 'pre-antibiotic era' when infectious diseases caused nearly one-third of all reported deaths may return. The gram-positive bacterium methicillin-resistant *Staphylococcus aureus* (MRSA) is a prototypical multidrug-resistant organism listed by the Centers for Disease Control (CDC) as a top priority pathogen. *S. aureus* is both a commensal microbe found in the nasal mucosa of ~30% of healthy adults and a human pathogen. Infections with *S. aureus* typically occur in immunocompromised individuals with underlying diseases-such as diabetes, acquired immunodeficiency syndrome or loss of neutrophil function-following disruption of the host's cutaneous or mucosal barriers. Disruption of these barriers can be caused by injury, surgical procedures, medical devices, and drug use which can lead to a litany of symptoms, including sepsis, severe skin infections, catheter-associated urinary tract infections and pneumonia.

In 2017 alone, severe cases of MRSA led to an estimated 119,000 systemic infections with a mortality rate of 17%. While MRSA has historically been recognized for its role in healthcare-associated (HA) infections, community-associated (CA) infections have become more prevalent in the past 40 years and often coincide with worse health outcomes.

MRSA was first reported in 1961, only one year after the introduction of the anti-staphylococcal penicillin known as methicillin into clinical practice. β-Lactam resistance in MRSA is due to expression of the altered penicillin-binding protein PBP2a, which is only weakly inhibited by virtually all β-lactam antibiotics. PBP2a is encoded by *mecA* or similar homologues that are part of a mobile genetic element called the staphylococcal cassette chromosome *mec* (SCCmec)*,* which can be further classified into fourteen types (I-XIV). *SCCmec* types I, II, and III are commonly found in healthcare-associated MRSA (HA-MRSA) while *SCCmec* IV and V are found in both HA-MRSA and community-associated MRSA (CA-MRSA). The different *SCCmec* types contain other genetic elements that confer resistance to many classes of antibacterial drugs such as tetracyclines, glycopeptides, lipopeptides, macrolides, and aminoglycosides.

Despite the growing rise of antimicrobial resistance, there have only been six new first-in-class antibacterial drugs approved in the past 20 years. Clinicians continue to rely almost exclusively on intravenously administered vancomycin for treatment of hospitalized patients with serious MRSA infections while intravenous daptomycin is used for MRSA bacteremia and endocarditis. Linezolid is an attractive oral switch therapy for MRSA infections and is widely used for treatment of pneumonia and skin and soft tissue infections. Resistance to all three agents has been reported. The limited treatment options, inadequate number of antibacterial agents in the drug pipeline, and emerging resistance to standard-of-care treatment options all point to the need for novel therapeutics with unconventional mechanisms of action.

The bacterial membrane has traditionally been overlooked in antibacterial drug research because membrane-targeting agents are generally considered poorly selective. However, selectivity can be achieved by binding prokaryotic structural lipids, membrane proteins, and cell wall components enabling discrimination from host cell membranes.

Bacterial membranes represent particularly promising antibacterial targets since they are essential under replicating and non-replicating conditions as well as in planktonic and biofilm cultures. Moreover, the development of resistance to compounds targeting the bacterial membrane is more difficult than to classical antibiotics against easily mutable proteins. The naturally occurring cationic antimicrobial peptides (CAMPs) that disrupt bacterial membranes are part of prokaryotes' and eukaryotes' innate immune system while several classes of Federal Drug Administration (FDA)-approved antibiotics exert their activity through bacterial membrane disruption including the polymyxins, bacitracins, lipopeptides, and lipoglycopeptides **(****FIG. 2****).**

The potent antibacterial activity of the synthetic retinoids CD437 and CD1530 **(****FIG. 2****)** was recently shown to be caused by membrane disruption as the primary mechanism of action. These aforementioned membrane-targeting antibacterial agents are noted for their poor pharmacokinetic behavior and/or toxicity which emanates from their amphipathic nature and undesirable physicochemical properties. Herein, Applicants report their investigation of a membrane-disrupting aminoquinoline antibacterial scaffold that led to the identification of a highly potent and selective gram-positive antibacterial agent with attractive physicochemical properties.

### Example 1.2. Design and Synthesis of Anti-MRSA Compounds

Applicants previously reported the identification of the 4-quinolinol derivative DNAC-2 from a high-throughput screening (HTS) campaign with moderate activity (MIC = 8 µg/mL) against MRSA **(****FIG. 3****).** Intriguingly, DNAC-2 was found to target the membrane of gram-positive bacteria resulting in partial membrane depolarization while displaying no toxicity towards eukaryotic membranes. In addition to DNAC-2, a few other substituted quinolines were identified with the same mechanism of action typified by quinoline **1 (****FIG. 3****)** indicating flexibility at the 4-position. Applicants were attracted to the 4-substituted quinoline scaffold based on its promising activity, chemical tractability for analog synthesis, and prevalence in several approved drugs. Applicants initially sought to examine the structure-activity relationships (SAR) of **1** (not of the invention) through substitution and replacement of the 4-aryl ring with more polar and non-planar substituents **(****FIG. 3****).** In parallel, Applicants wanted to explore modification and substitution to the quinoline heterocycle through introduction of nitrogen atoms and introduction of more polar substituents at the 2-, 7- and 8-positions to decrease the lipophilicity.

### Example 1.3. Chemistry

The first series of quinoline analogues were synthesized from a common set of quinolone building blocks **2-10 (4, 5** are not of the invention) that were prepared via a modified Conrad-Limpach reaction between substituted aniline derivatives and 4,4,4-trifluoroacetoacetate in neat polyphosphoric acid (PPA) at 120 °C. *Meta*-substituted anilines typically formed a mixture of both the 5- and 7-regioisomers that were challenging to separate and led to reduced isolated yields of the desired 7-regioisomers, whereas *ortho*-substituted anilines exclusively afforded the 8-regioisomeric products. The quinolones were evaluated for antibacterial activity and only compounds **3-6** and **10** (DNAC-2 is the same as **6)** were found to be active **(Table 1).** Consequently, only these compounds were derivatized by introduction of a substituent at the 4-position. Quinolone **3** was converted to the corresponding triflate **3a** (not of the invention) employing triflic anhydride and reacted with various amines and phenols by nucleophilic aromatic substitution (S_{N}Ar) to afford **13, 15-22, 25-29, 44, 53-54, 59-62,** and **80-83** (not of the invention). This strategy proved less effective for electron-deficient amines as well as quinolones with electron donating substituents. In these cases, Applicants utilized a complimentary route by conversion of quinolones to the corresponding aryl chloride or aryl bromides **3b-6b** and **10b** (not of the invention) followed by Buchwald-Hartwig amination to provide **2, 12, 14, 23-24, 30-38, 42-43, 45-51, 55-58, 63-64** and **84** (of these, only **2** is of the invention). 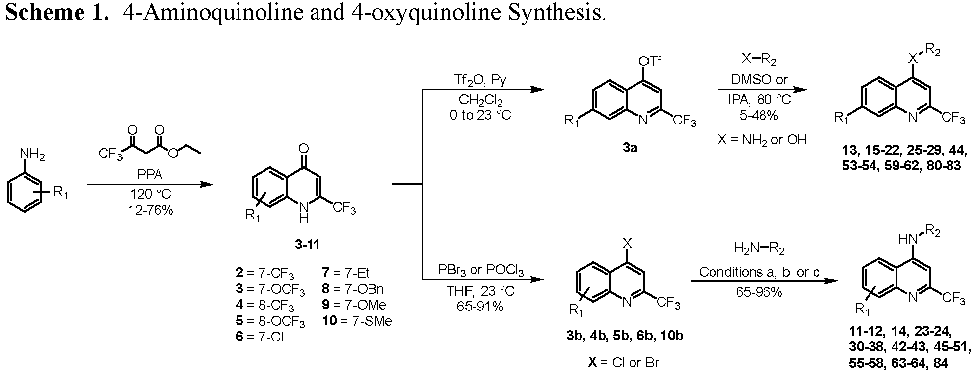 Conditions: *^{a}*K₃PO_{4'} SPhos; Pd₂(dba)₃·CH₂Cl₂, THF, 55 °C; ^{b} *t*-BuONa, DPPF, Pd(dppf)Cl₂·CH₂Cl₂, THF, 55 °C; and ^{c} *t*-BuONa, XantPhos, Pd₂(dba)₃, dioxane 80 °C.

Compounds containing a difluoromethyl C-2 substituent were prepared analogously by Conrad-Limpach reaction between 3-trifluoromethylaniline and 4,4-difluoroacetoacetate to afford quinolone **67** (not of the invention), which was activated by triflic anhydride to **67a** (not of the invention) and elaborated to **68** (not of the invention) and **69** (not of the invention) by S_{N}Ar substitution with 3,4-dichloroaniline and 3-trifluormethoxyaniline, respectively **(Scheme 2).** Analogs containing a dimethylaminosulfonyl C-7 substituent could not be synthesized using the usual PPA-mediated procedure and required substantially more thermal energy. Compound **66** was instead prepared by refluxing 3-(dimethylaminosulfonyl)aniline and 4,4,4-trifluoroacetoacetate at 255 °C in diphenyl ether **(Scheme 2).** Chlorination of **66** with POCl₃ yielded **66a** (not of the invention) that was diversified to **39-41** (not of the invention) by Buchwald-Hartwig amination. 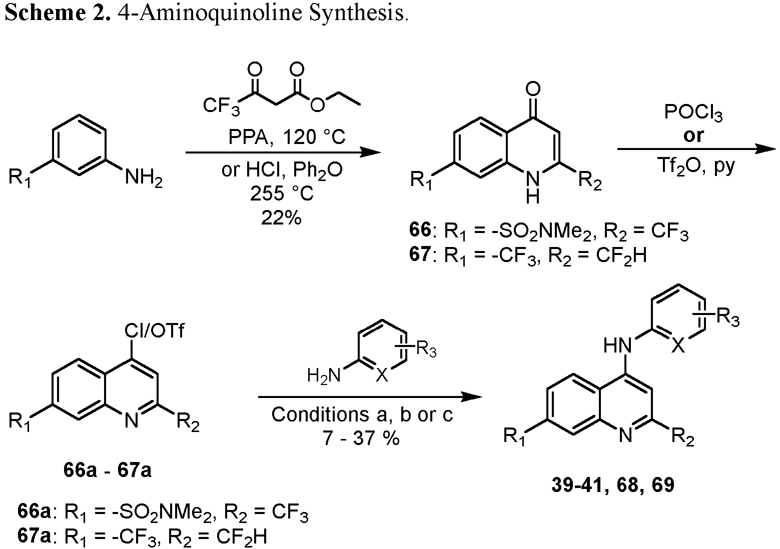 Conditions: ^{a} DMSO, 80 °C; and ^{b} HCl, EtOH, reflux ^{c}Pd₂(dba)₃, XantPhos, *t*-BuONa, dioxane 80 °C.

Applicants synthesized a series of mono-, di- and tri-fluorinated analogs of the B-ring in an attempt to replace the lipophilic C-7 trifluoromethyl group. While *m*-fluoroaniline reacted with 4,4,4-trifluoroacetoacetate in neat polyphosphoric acid (PPA) at 100 °C to furnish **70,** the di- and tri-fluoroanilines required heating at 150 °C to effect cyclization to quinolones **71-73** (not of the invention) **(Scheme 3).** Halogenation of **70-73** to quinolines **70a-73a** (not of the invention) followed by Buchwald-Hartwig amination as described previously yielded **74-79** (not of the invention).

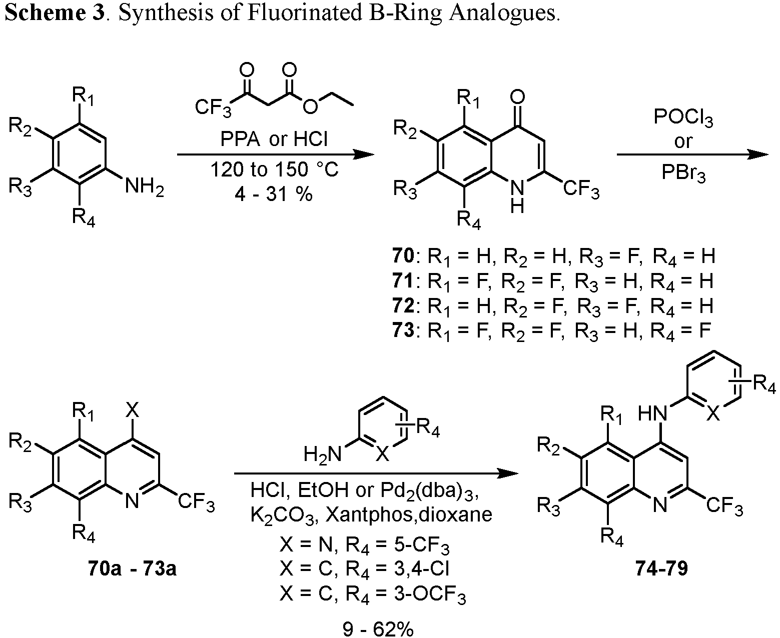

In an attempt to reduce the lipophilicity of the aminoquinolines, Applicants targeted the synthesis of quinazolines containing an additional nitrogen atom in the A-ring. Synthesis commenced from commercially available 2-amino-4-(trifluoromethyl)benzonitrile that was oxidized from the nitrile to the amide intermediate **86** by treatment with an alkaline solution of hydrogen peroxide **(Scheme 4).** Treatment of the resulting substituted aniline **86** with 2,2,2-trifluoroacetyl chloride furnished the bis-amide intermediate **87,** which was cyclized to the quinazolone **88** (not of the invention) employing potassium hydroxide in ethanol at reflux. Chlorination of quinazolone **88** with thionyl chloride in DMF followed by S_{N}Ar substitution with 3,4-dichloroaniline, 3-trifluoromethoxyaniline, and 2-amino-5-trifluoromethylpyridine afforded the final 4-aminoquinazolines **89-91** (not of the invention).

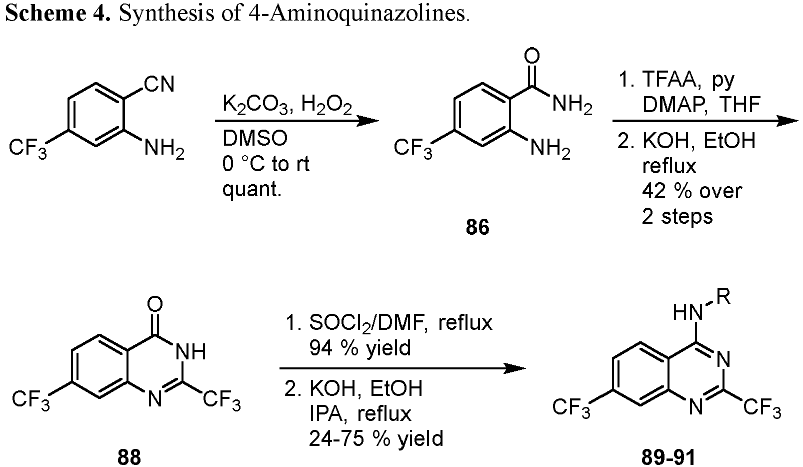

The pyrido[2,3-*d*]pyrimidine scaffold containing two additional nitrogen atoms was investigated as a quinoline isostere in an attempt to further decrease lipophilicity. The synthesis began by S_{N}Ar substitution of 2-chloro-3-cyano-6-trifluoromethylpyridine with ammonia in THF followed by base-promoted hydration of the cyano group to furnish amide **92 (Scheme 5).** Subsequent condensation of **92** with ethyl trifluoroacetate and base-catalyzed annulation afforded an intermediate pyrido[2,3-*d*]pyrimidin-4-one derivative that was converted to **93** by POCl₃ mediated chlorination. S_{N}Ar substitution of **93** (not of the invention) with 3,4-dichloroaniline yielded **94** (not of the invention) while reaction of **93** with 2-amino-5-trifluoromethylpyridine to provide **95** (not of the invention) required the complimentary Buchwald-Hartwig amination.

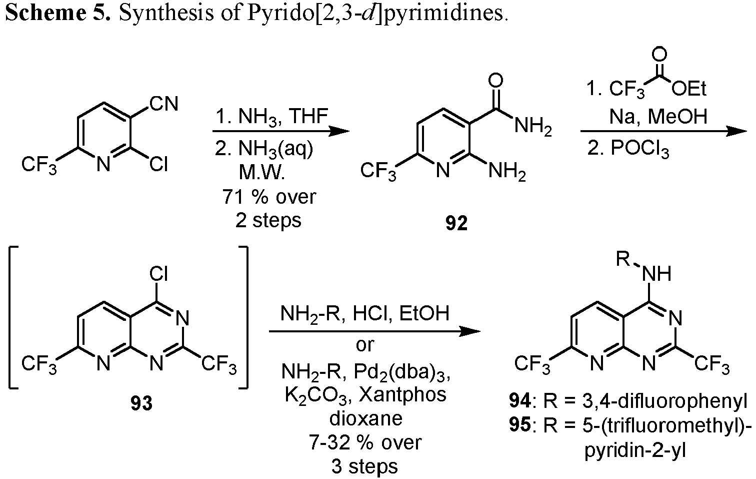

The cinnoline analogs were the final set of aza-analogs of the quinoline scaffold prepared. Synthesis of the requisite cinnoline-4-one building block was achieved by diazotization of 2-amino-4-trifluoromethylacetophenone **96** with sodium nitrite and aqueous hydrogen chloride. Following removal of the solvents *in vacuo,* the resultant diazonium ion was directly treated with sodium acetate at 100 °C to afford **97 (Scheme 6)**. The cinnoline-4-one intermediate **97** was then activated with phosphorus oxychloride and coupled to 3,4-difluoroaniline and 2-amino-5-trifluoromethylpyridine by S_{N}Ar substitution and Buchwald-Hartwig reaction to provide **98** and **99** (not of the invention).

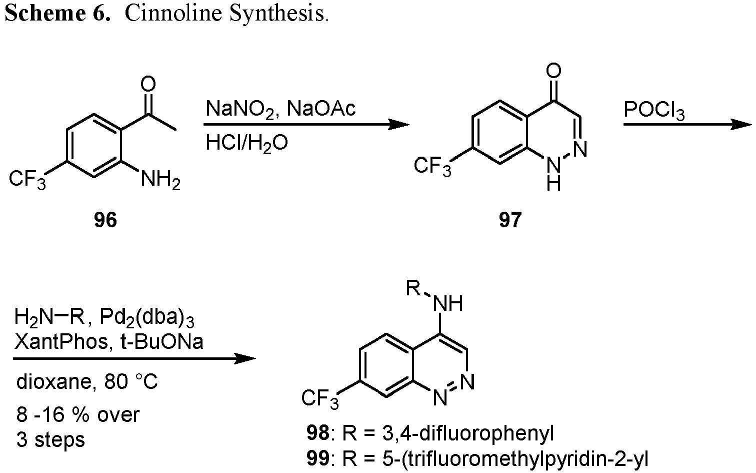

Applicants developed an alternate quinoline synthesis to explore modification of the C-2 position featuring a 2,4-dichloroquinoline intermediate. This was accomplished by intramolecular Claisen-like condensation of ethyl *N*-acetyl-2-amino-4-trifluoromethylbenzoate mediated by potassium bis(trimethylsilyl)amide (KHMDS) to afford a 4-hydroxyquinoline-(2*H*)-one intermediate, which was converted to 2,4-dichloroquinoline **100** (not of the invention) by refluxing in phosphorus oxychloride. S_{N}Ar substitution by Boc-protected piperazine gave **101** along with the C-4 regioisomer (not shown). Buchwald-Hartwig coupling of **101** (not of the invention) and 2-amino-5-trifluoromethylpyridine followed by TFA deprotection of the Boc group gave the desired analogue **102** (not of the invention). The regioisomeric analog **103** (not of the invention) was prepared from the corresponding C-4 piperazine intermediate isolated as a side-product in the preparation of **101.** The C-2 and C-4 morpholino substituted analogues **104** (not of the invention) and **105** (not of the invention) were synthesized in an analogous fashion.

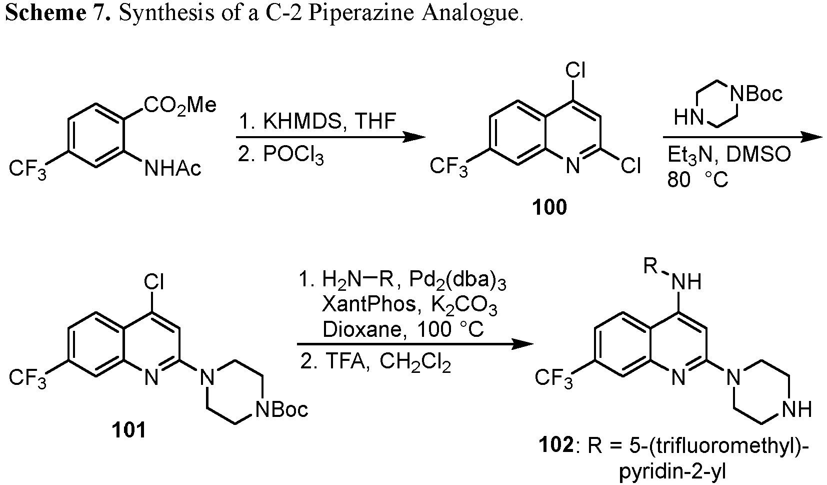

Applicants next conceived of a hybrid scaffold of the initial active quinoline-4-ones and the aryl-substituted 4-aminoquinolines, giving an aryl-substituted 5-aminoquinoline-4-ones scaffold **(Scheme 8).** The two-step synthesis started from condensation of 3-bromo-5-trifluoromethylaniline and ethyl 2,2,2-trifluoromethylacetoacetate in neat PPA to give two separable regioisomers **106** and **107 (107** not of the invention). The structure of the regioisomers were assigned by ¹H NMR analysis of the debrominated products generated by palladium-catalyzed dehalogenation (not shown). Following optimization of the Buchwald-Hartwig amination conditions, Applicants were able to access the desired C-5 substituted aminoquinoline-4-ones **108-118** from **106.** The corresponding C-7 substituted aminoquinoline-4-ones **119-122** were prepared from 107. The C-7 fluoro derivative 123 was synthesized analogously from 3-bromo-5-fluoroaniline.

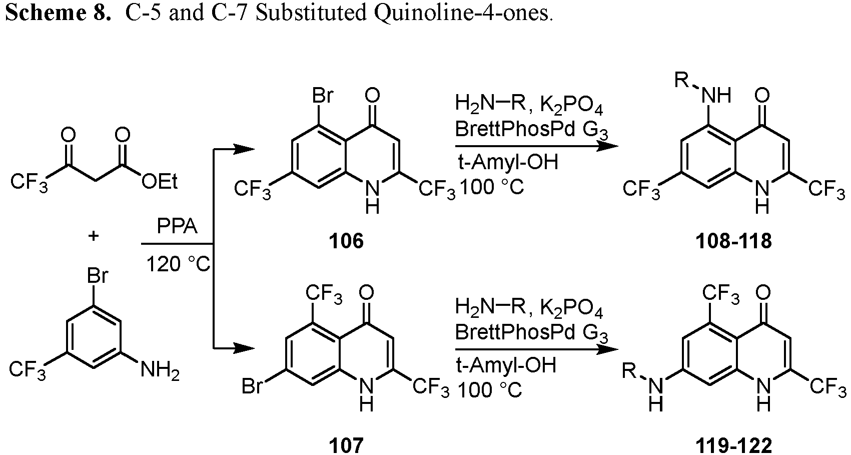

Based on the promising activity of quinolin-4-one **111,** but low solubility **(Table 8),** Applicants sought to prepare an alkoxycarbonate prodrug for in vivo studies to increase solubility and bioavailability based on the precedent of previous literature, which successfully applied this strategy in preclinical development of novel antimalarial quinolone ELQ-330. Direct installation of the promoiety onto **111** was unsuccessful; however, the alkoxycarbonate promoiety could be introduced in onto 5-bromoquinoline **106** by cesium carbonate mediated alkylation of chloromethyl ethyl carbonate in acetone. The resulting methyloxycarbonate ester **124** (not of the invention) was then converted to the desired final product **125** (not of the invention) via a Buchwald-Hartwig amination with 2-amino-5-trifluoromethylpyridine.

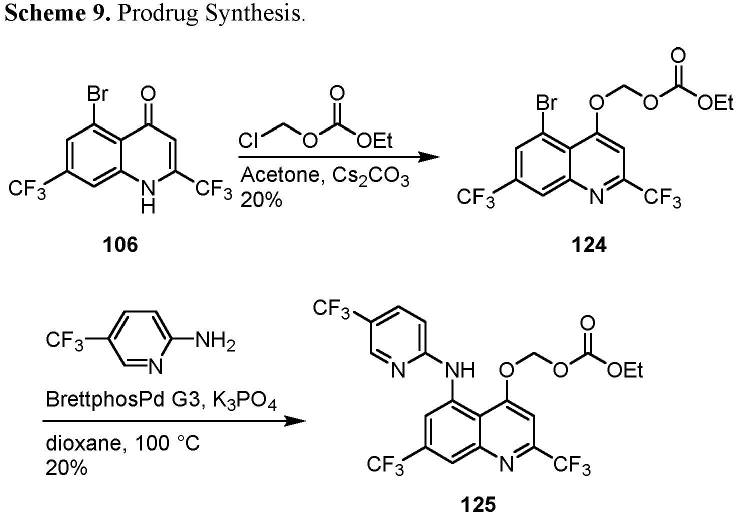

### Example 1.4. Microbiology

The antibacterial activity of compounds was initially determined against a clinical strain of methicillin-resistant *S. aureus* (FPR3757) in Muellar-Hinton (MH) broth according to CLSI guidelines to determine the minimum inhibitory concentration (MIC) that resulted in complete inhibition of observable growth. The first set of compounds evaluated were analogs at the C-7 and C-8 positions of the initial quinolone hit **DNAC-2** since the other HTS hits identified (data not shown) were substituted at these positions **(Table 1).** Applicants first explored modification at the C-7 position with a small series of electron-donating and withdrawing substituents. The trifluoromethyl **2** and trifluoromethoxy **3** are the most potent with MICs of 4-8 µg/mL while chloro **6** is slightly weaker with an MIC of 20 µg/mL. However, analogs containing electron donating groups at C-7 including ethyl **7,** benzyloxy **8,** methoxy **9** and methylthio **10** are weakly active displaying MICs of 320-640 µg/mL indicating electron-donating substituents at C-7 are poorly tolerated. The impact of electronics is best illustrated with methoxy **9,** which is 80-160 less potent that the isosteric trifluoromethoxy **3.** The C-8 position was evaluated with trifluoromethyl **4** and trifluoromethoxy **5** containing the optimal C-7 substituents. Both **4** and **5** (not of the invention) are equipotent to the corresponding C-7 analogs **2** and **3** indicating some flexibility of the quinolone scaffold.

**Table 1. Substituted Quinoline-4-one Analogues (* = not of the invention).**

| 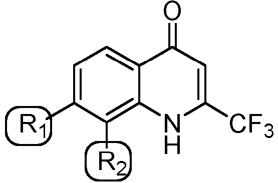 | | | | |
|---|---|---|---|---|
| **Compound** | **R₁** | **R₂** | **MIC µg/mL** | **cLogP** |
| **2** | CF₃ | H | 5 | 2.2 |
| **3** | OCF₃ | H | 4-8 | 2.4 |
| **4*** | H | CF₃ | 2-4 | 2.2 |
| **5*** | H | OCF₃ | 5 | 2.4 |
| **6** | chloro | H | 20 | 2.0 |
| **7** | ethyl | H | 320 | 2.3 |
| **8** | benzylether | H | 640 | 3.1 |
| **9** | methoxy | H | 640 | 1.3 |
| **10** | thiomethyl | H | 640 | 1.9 |

Applicants next explored the SAR of the C-4 aryl substituent of 4-aminoquinoline **1** whose MIC is 8 µg/mL **(Table 2).** Applicants' first series of compounds contain a 7-trifluoromethyl rather than the 8-trifluoromethoxy substituent found in **1.** The 2'-, 3'-, and 4'-chlorophenyl analogs **15-17** (not of the invention) helped to define the steric requirements for activity: 2'-chlorophenyl **15** is inactive while both 3'-chlorophenyl **16** and 4'-chlorophenyl **17** possess MICs of 0.125-0.25 µg/mL, which represents a dramatic 32-64-fold increase in potency over **1.** Given the enhanced potency of the chloro-substituted analogs, Applicants conducted a halogen scan and evaluated 3'-fluorophenyl **18** (not of the invention), 3'-bromophenyl **19** (not of the invention)and 3'-iodophenyl **20** (not of the invention). The more lipophilic halogens **19** and **20** maintain potent activity with MICs of 0.25 µg/mL while the fluoro analog has a substantial 16-64-fold loss of potency. Applicants also explored a couple of 3',4'-disubstituted analogs with 3',4'-fluorophenyl **21** (not of the invention) and 3',4'-dichlorophenyl **22** (not of the invention). Both analogs display further improvements in potency relative to the corresponding mono-halogenated analogs and the MIC of 3',4'-dichlorophenyl **22** decreased to 0.0625 µg/mL, the lowest value among the series of analogs described in Table 2. Additionally, a broader array of substituents were explored at the 3'- and 4'-positions of the aryl ring including phenyl **11,** 3'-acetyl **13,** 3'-cyanophenyl **14,** 3'-hydroxymethylphenyl **23,** 3'-methylthiophenyl **24,** 3'-methoxyphenyl **25,** 3'-trifluoromethylphenyl **26,** 3'-trifluoromethoxyphenyl **27,** 3'-(morpholino)phenyl **29,** 4'-(morpholino)phenyl **30** and 3',4'-(methylenedioxy)phenyl **28** (all not of the invention). Analogs containing polar acetyl, cyano, methoxy, hydroxymethyl, methylenedioxy, and morpholino substituents are inactive or weakly active with MICs generally >16 µg/mL. By contrast, analogs containing lipophilic groups including methylthio and trifluoromethyl are potent with MICs of 0.25-0.50 µg/mL, the exception being trifluoromethoxy **27,** whose MIC is 8 µg/mL. In an attempt to decrease the lipophilicity, Applicants replaced the phenyl ring of 3'-trifluoromethylphenyl **26** by a pyridine to furnish 5'-(trifluoromethyl)pyridin-2-yl **31** (not of the invention), which fortuitously maintains activity providing an identical MIC to **26** while decreasing the calculated logP by 1.2 units to 6.0. While introduction of an appropriately substituted arylamino group led to a substantial enhancement in activity relative to the simple quinolones shown in **Table 1,** this boost in potency came at the expense of substantially increased lipophilicity. This is exemplified by 3'-trifluoromethylphenyl **26** whose 16-fold improvement in potency relative to the parent quinolone **3** is offset by a 5.1 unit increase in the calculated logP. Attempts to decrease lipophilicity by introduction of polar substituents onto the aryl ring led to sharp reductions in potency. However, heterocyclic replacement of the phenyl ring by a pyridine is tolerated providing a means to partially address the increased lipophilicity of the *N-*(arylamino)quinolines.

The SAR of the *N*-(arylamino)quinoline scaffold was further probed at the C-7 and C-8 positions with 7-methoxy, 7-trifluoromethoxy, 7-(dimethylamino)sulfonyl, 8-trifluoromethyl, and 8-trifluoromethoxy substituents with the C-4 aryl moiety selected from representatives of **12-31** (not of the invention) including 3',4'-difluorophenyl **21** (MIC of 0.125-0.25 µg/mL), 3',4'-dichlorophenyl **22** (MIC of 0.0625 µg/mL), 3'-(trifluoromethoxy)phenyl **27** (MIC of 8 µg/mL), and 5'-(trifluoromethyl)pyridin-2-yl **31** (MIC of 0.25 µg/mL). Replacement of the C-7 trifluoromethyl group by a trifluoromethoxy group in **32-34** (not of the invention) yielded flat SAR with MICs ranging from 0.5-1 µg/mL. The SAR trend from this limited set of compounds did not parallel the SAR observed with the 7-trifluoromethyl series of compounds. The isosteric 7-methoxy analogs **35-38** (not of the invention) were largely inactive (MICs of ≥ 32 µg/mL), a result consistent with the quinolone SAR described in **Table 1.** The observation that electron-withdrawing substituents at C-7 are favorable prompted exploration of the 7-dimethylaminosulfonyl group with **39-41** (not of the invention) since sulfonamides are electron-withdrawing and considerably more polar than a trifluoromethyl group. Unfortunately, this set of compounds was only weakly active with MICs ranging from 8 to >32 µg/mL suggesting optimal quinoline substituents at C-7 should not only be electron-withdrawing, but also nonpolar. Analogs bearing trifluoromethyl and trifluoromethoxy substituents at C-8 exhibited remarkably flat SAR with MICs of 0.5-2.0 µg/mL. The SAR trend was inconsistent with the 7-trifluoromethyl substituted analogs, whose MICs varied over 128-fold for the same set of C-4 aryl substituents. Taken together, the SAR from **32-47** (not of the invention) demonstrates substitution at C-7 is preferred and optimal substituents at this position should be non-polar and strongly electron-withdrawing.

**Table 2. Aryl 4-aminoquinoline Analogues (not of the invention).**

| | | | | | |
|---|---|---|---|---|---|
| 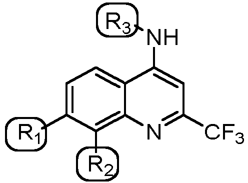 | | | | | |

| **Compound** | **R₁** | **R₂** | **R₃** | **MIC µg/mL** | **cLogP** |
|---|---|---|---|---|---|
| **1** | H | OCF₃ | 3-(trifluoromethoxy)phenyl | 8 | 7.9 |
| **11** | CF₃ | H | phenyl | 128 | 6.4 |
| **12** | CF₃ | H | 2-isopropylphenyl | 320 | 7.8 |
| **13** | CF₃ | H | 3-acetylphenyl | >128 | 5.9 |
| **14** | CF₃ | H | 3-benzonitrile | >32 | 5.9 |
| **15** | CF₃ | H | 2-chlorophenyl | >16 | 7.1 |
| **16** | CF₃ | H | 3-chlorophenyl | 0.25 | 7.1 |
| **17** | CF₃ | H | 4-chlorophenyl | 0.12 | 7.1 |
| **18** | CF₃ | H | 3-fluorophenyl | 4-16 | 6.5 |
| **19** | CF₃ | H | 3-bromophenyl | 0.25 | 7.3 |
| **20** | CF₃ | H | 3-iodophenyl | 0.25 | 7.5 |
| **21** | CF₃ | H | 3,4-difluorophenyl | 0.12-0.25 | 6.6 |
| **22** | CF₃ | H | 3,4-dichloropheyl | 0.06 | 7.7 |
| **23** | CF₃ | H | 3-(hydroxymethyl)phenyl | >16 | 5.3 |
| **24** | CF₃ | H | 3-thiomethylphenyl | 0.25-0.5 | 7.0 |
| **25** | CF₃ | H | 3-methoxyphenyl | >64 | 6.3 |
| **26** | CF₃ | H | 3-(trifluoromethyl)phenyl | 0.25 | 7.3 |
| **27** | CF₃ | H | 3-(trifluoromethoxy)phenyl | 8 | 7.4 |
| **28** | CF₃ | H | 3,4-(methylenedioxy)phenyl | >4 | 6.2 |
| **29** | CF₃ | H | 3-(morpholino)phenyl | >16 | 5.8 |
| **30** | CF₃ | H | 4-(morpholino)phenyl | >16 | 5.8 |
| **31** | CF₃ | H | 5-(trifluoromethyl)pyridin-2-yl | 0.25 | 6.1 |
| **32** | OCF₃ | H | 3,4-dichlorophenyl | 0.5-1 | 8.2 |
| **33** | OCF₃ | H | 3-(trifluoromethoxy)phenyl | 1 | 7.9 |
| **34** | OCF₃ | H | 5-(trifluoromethyl)pyridin-2-yl | 1 | 6.5 |
| **35** | OMe | H | 3,4-dichlorophenyl | 2 | 6.0 |
| **36** | OMe | H | 3,4-difluorophenyl | >32 | 4.9 |
| **37** | OMe | H | 3-(trifluoromethoxy)phenyl | >32 | 5.7 |
| **38** | OMe | H | 5-(trifluoromethyl)pyridin-2-yl | 32 | 4.3 |
| **39** | S(O)₂N(Me | H | 3,4-difluorophenyl | >32 | 5.1 |
| **40** | S(O)₂N(Me | H | 3-(trifluoromethoxy)phenyl | 8 | 5.9 |
| **41** | S(O)₂N(Me | H | 5-(trifluoromethyl)pyridin-2-yl | 8 | 4.5 |
| **42** | H | OCF₃ | 3,4-dichlorophenyl | 0.5 | 8.2 |
| **43** | H | OCF₃ | 3-(trifluoromethoxy)phenyl | 1 | 7.8 |
| **44** | H | OCF₃ | 5-(trifluoromethyl)pyridin-2-yl | 1 | 6.5 |
| **45** | H | CF₃ | 3,4-dichlorophenyl | 0.5-2 | 8.2 |
| **46** | H | CF₃ | 3-(trifluoromethoxy)phenyl | 1 | 7.9 |
| **47** | H | CF₃ | 5-(trifluoromethyl)pyridin-2-yl | 0.5 | 6.5 |

The promising activity of compound **31** containing a 5'-(trifluoromethyl)pyridin-2-yl-amino moiety appended to C-4 of the quinoline prompted Applicants to explore more diverse heterocyclic substituents at C-4 **(Table 3).** A primary objective in these analogues was to decrease the overall lipophilicity through introduction of polar atoms and to reduce the planarity by increasing the sp³ character since lipophilic and planar molecules tend to have poor solubility that adversely impacts drug disposition properties. Replacement of the 5'-(trifluoromethyl)pyridin-3-yl-amino group at C-4 with a closely related 4'-(chloro)pyridin-2-yl-amino group in **48** (not of the invention) led to an 8-fold loss of activity while transposition of the pyridine nitrogen by one atom in 2'-(trifluoromethyl)pyridin-5-yl-amino **49** (not of the invention) completely abolished activity. These findings foreshadowed Applicants' unsuccessful attempts to modify the C-4 substituent. Thus pyridones **50-52,** pyridine **53,** picolinate **54,** pyrimidine **56,** cyclohexane **57,** hydroxypiperidine **58,** morpholine **59,** aminomethylpyrrolidine **60,** indazole **61,** isoindole **62,** pyrrolopyridine **63,** indolone **64** and azabicyclooctanol **65** were inactive at the highest concentration evaluated (MIC > 32 µg/mL) (all not of the invention). Only, aminothiazole **55** (not of the invention) demonstrated moderate activity with an MIC of 2 µg/mL.

**Table 3. Heterocyclic 4-substitutions (not of the invention).**

| 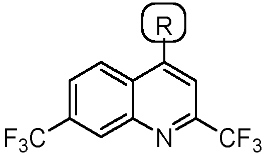 | | | | | | | |
|---|---|---|---|---|---|---|---|
| **Compound** | **R** | **MIC µg/mL** | **cLogP** | **Compound** | **R** | **MIC µg/mL** | **cLogP** |
| **31** | 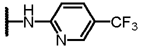 | 0.25 | 6.0 | **57** | 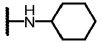 | >16 | 6.6 |
| **48** | 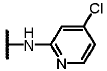 | 2 | 5.8 | **58** | 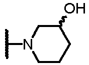 | >32 | 4.0 |
| **49** | 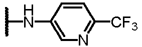 | >32 | 6.0 | **59** | 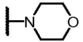 | >32 | 4.0 |
| **50** | 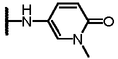 | >32 | 4.2 | **60** | 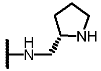 | >32 | 4.9 |
| **51** | 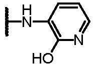 | >128 | 5.4 | **61** | 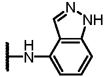 | >32 | 6.0 |
| **52** | 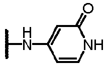 | >32 | 3.7 | **62** | 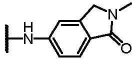 | >32 | 5.2 |
| **53** | 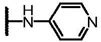 | >32 | 5.1 | **63** | 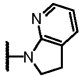 | >32 | 5.3 |
| **54** | 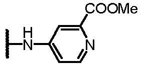 | >32 | 4.7 | **64** | 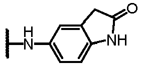 | >32 | 5.0 |
| **55** | 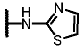 | 1-2 | 4.9 | **65** | 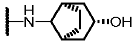 | >32 | 5.3 |
| **56** | 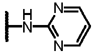 | >32 | 4.2 | | | | |

Further structural modifications were focused on reducing the calculated LogP by modifications of the quinoline core employing the optimal C-4 substituents: 3',4'-dichlorophenyl, 3'-(trifluoromethoxy)phenyl and 5'-(trifluoromethyl)pyridin-2-yl from compounds **22, 27,** and **31,** respectively. The trifluoromethyl groups at the C-2 and C-7 positions contribute significantly to the overall lipophilicity, thus the next series of analogs explored replacement of the trifluoromethyl group by difluoromethyl and aryl fluorides, which were predicted to lower the logP by approximately 0.7 units per trifluoromethyl group **(Table 4).** Replacement of the C-2 trifluoromethyl group of **22** with a difluoromethyl group afforded compound **68** (not of the invention), which is 16-fold less potent than **22.** Conversely, compound **69** (not of the invention) exhibits a 16-fold increase in potency relative to the corresponding trifluoromethyl analog **27.** Applicants cannot reconcile the disparate impact on potency of the difluoromethyl group based on this limited set of analogs, but the difluoromethyl group appears to level the SAR as both **68** and **69** have similar MICs. Replacement of the C-7 trifluoromethyl group by a fluorine was explored with analogs **74-79** (not of the invention). Substitution of the C-7 trifluoromethyl group by a 7-fluoro moiety in **74-76** led to uniform 4-8-fold reductions in potency relative to the corresponding trifluoromethyl analogs **22, 27** and **31** providing MICs ranging from 0.5-2.0 µg/mL. Given the more predictable SAR of the aryl fluoride analogs, Applicants sought to introduce additional fluorine atoms in the B-ring of **76** at the 5, 6, and 8-positions with difluorinated analogs **77-78** and trifluorinated analog **79.** While fluorine was poorly tolerated at the 5 and 6-positions, the 6,7-difluoro analog **78** fully regained the activity of the parent trifluoromethyl analog **31.** Collectively, these results indicate modest attenuation of the logP can be achieved by replacement of the lipophilic trifluoromethyl groups with fluorine atoms while maintaining potent activity.

**Table 4. Fluorine Substitutions (not of the invention).**

| 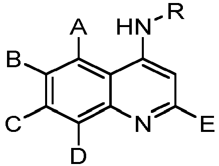 | | | | | | | | |
|---|---|---|---|---|---|---|---|---|
| **Compound** | **A** | **B** | **C** | **D** | **E** | **R** | **MIC µg/mL** | **cLogP** |
| **68** | H | H | CF₃ | H | CHF₂ | 3,4-dichlorophenyl | 1 | 6.3 |
| **69** | H | H | CF₃ | H | CHF₂ | 3-(trifluoromethoxy)phenyl | 0.5 | 6.6 |
| **74** | H | H | F | H | CF₃ | 3,4-dichlorophenyl | 0.5 | 7.0 |
| **75** | H | H | F | H | CF₃ | 3-(trifluoromethoxy)phenyl | 2 | 6.7 |
| **76** | H | H | F | H | CF₃ | 5-(trifluoromethyl)pyridin-2-yl | 1 | 5.3 |
| **77** | F | F | H | H | CF₃ | 5-(trifluoromethyl)pyridin-2-yl | 16-32 | 5.3 |
| **78** | H | F | F | H | CF₃ | 5-(trifluoromethyl)pyridin-2-yl | 0.25 | 5.3 |
| **79** | F | F | H | F | CF₃ | 5-(trifluoromethyl)pyridin-2-yl | >32 | 5.5 |

With extensive coverage of the C2, C-4, C-7 and C-8 positions of the 4-aminoquinoline, the SAR campaign moved towards heteroatom modifications of the 4-aminoquinoline core. Applicants first studied the importance of the 4-amino group and specifically the importance of an H-bond donor at this position with ether analogues **80-83** (not of the invention), *N*-methyl derivative **84** (not of the invention) and amide **85** (not of the invention) **(Table 5).** Compounds **80-85** are inactive with MICs greater than 32 µg/mL indicating an NH moiety is useful for activity and a one atom linker is often preferred. Applicants then explored quinazoline analogs **89-91** (not of the invention) containing a single aza substitution at the C-3 position, which retains a similar pharmacophore while lowering the calculated LogP by 1.5 units. The 3',4'-dichlorophenyl **89,** and 5'-(trifluoromethyl)pyridin-2-yl **91** quinazoline analogues are 8-fold less active than the parent quinolines **22, 31** while the trifluoromethoxy **90** derivative has an opposite 8-fold increase in potency relative to the parent quinoline **27.** The aza substitution thus appears to flatten the SAR as the potency of **89-91** varies only 4-fold from 0.5-2.0 µg/mL. Introduction of another nitrogen atom into the quinazoline at the C-8 position led to pyridopyrimidine derivatives **94-95** (not of the invention) and an attendant decrease in logP by almost 3 units. Unfortunately, both pyridopyrimidines **94** and **95** have drastically reduced activity with MICs ≥ 32 µg/mL.

**Table 5. Heteroatom Exchanges (not of the invention)**

| 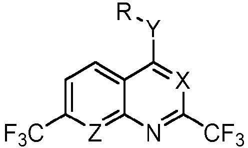 | | | | | | |
|---|---|---|---|---|---|---|
| **Compound** | **X** | **Y** | **Z** | **R** | **MIC µg/mL** | **cLogP** |
| **80** | CH | O | CH | 3-chlorophenyl | >32 | 6.8 |
| **81** | CH | O | CH | 3-fluorophenyl | >32 | 6.2 |
| **82** | CH | O | CH | 3,4-dichlorophenyl | >32 | 7.4 |
| **83** | CH | O | CH | 3-(trifluoromethoxy)phenyl | >32 | 7.1 |
| **84** | CH | NMe | CH | 4-trifluoromethylphenyl | >32 | 7.4 |
| **85** | CH | N(C=O) | CH | 5-(trifluoromethyl)pyridin-2-yl | >32 | 5.3 |
| **89** | N | NH | CH | 3,4-dichlorophenyl | 0.5 | 6.9 |
| **90** | N | NH | CH | 3-(trifluoromethoxy)phenyl | 1 | 6.6 |
| **91** | N | NH | CH | 5-(trifluoromethyl)pyridin-2-yl | 2 | 5.1 |
| **94** | N | NH | N | 3,4-difluorophenyl | 32 | 4.0 |
| **95** | N | NH | N | 5-(trifluoromethyl)pyridin-2-yl | >32 | 5.8 |

A few remaining miscellaneous modifications to reduce the logP of the 4-aminoquinoline scaffold are described in **Table 6** and **Table 7.** The cinnoline analogues **98** and **99** (not of the invention) lacking a C-2 trifluoromethyl group and containing a nitrogen atom at C-2 are unsurprisingly inactive **(Table 6).** Remarkably, introduction of a piperazine at C-2 with **102** (not of the invention) was reasonably well tolerated yielding an MIC of 0.5-1.0 µg/mL while the morpholine analogue **104** (not of the invention) is inactive **(Table 7).** The piperazine and morpholine constitutional isomers **103-104** (not of the invention) have modest activity with MICs of 4-8 µg/mL.

**Table 6. Cinnoline and Amide Analogues (not of the invention).**

| **Compound** | **Core Structure** | **R** | **MIC µg/mL** |
|---|---|---|---|
| **98** | 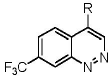 | 3,4-difluoroanilino | >32 |
| **99** | 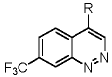 | 5-(trifluoromethyl) pyridin-2-yl-amino | >32 |

**Table 7. C-2 and C-4 Substituted Quinolines (not of the invention).**

| 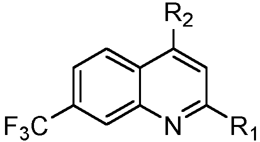 | | | | |
|---|---|---|---|---|
| **Compound** | **R₁** | **R₂** | **MIC µg/mL** | **cLogP** |
| **102** | Piperazinyl | 5-(trifluoromethyl)pyridin-2-yl-amino | 0.5-1 | 5.1 |
| **103** | 5-(trifluoromethyl)pyridin-2-yl-amino | piperazinyl | 4-8 | 5.1 |
| **104** | Morpholino | 5-(trifluoromethyl)pyridin-2-yl-amino | >32 | 5.1 |
| **105** | 5-(trifluoromethyl)pyridin-2-yl-amino | morpholino | 8 | 5.1 |

The final series of compounds investigated was a hybrid scaffold of the initial active quinol-4-one **(Table 1)** and the aryl-substituted 4-aminoquinoline **(Table 2)** to afford an aryl-substituted 5-aminoquinolin-4-one scaffold **(Table 8)** in an attempt to lower the calculated logP. All of the 5-aminoquinolin-4-ones **108-123** with the exception of **118** showed good to outstanding antibacterial activity with MICs ranging from <0.06 to 4 µg/mL while simultaneously decreasing the calculated LogP by two and up to five units. The optimal C-4 substituents in the 4-aminoquinoline series yielded extremely potent 5-aminoquinolone analogues **108-111** with MICs less than 0.06 µg/mL and attendant dramatic reductions in lipophilicity. The constitutional isomers **119-123** containing the arylamino substituents at the C-7 rather than the C-5 position found in **108-111** were substantially weaker with MICs ranging from 1-4 µg/mL, representing a 16- to greater than 64-fold loss in potency. Compound **111** was the first potent derivative synthesized with a calculated logP of less than four. Given the impressive activity of **108-111** Applicants sought to further examine closely related substituents containing polar substituents and/or greater sp3 character including 5-fluoropyridin-2-yl-amino **112,** 5-dimethylaminopyridin-2-yl-amino **113,** 6-(trifluoromethyl)pyridazin-3-yl-amino **114,** 3-(*N,N*-dimethylsulfonamide)pyridine-6-yl-amino **115,** 4-amino-1*H*-indazolyl **116,** 4-(trifluoromethyl)cyclohex-1-yl-amino **117,** and morpholino **118,** whose calculated logPs ranged from 2.0 to 3.9. The SAR exhibited substantially greater flexibility than observed in the 4-aminoquinoline series **(Table 4)** and many of these analogs including **113-116** had respectable MICs ranging from 1-2 µg/mL **(Table 8).** Some of the analogs were exceptionally potent including fluoropyridine analog **112** and 4-(trifluoromethyl)cyclohex-1-yl-amino **117** whose MICs range from less than 0.06 to 0.12 µg/mL. Lastly, Applicants prepared **123** incorporating a 7-fluoro substituent in place of the trifluoromethyl group of **111** in an attempt to further modulate the lipophilicity. Gratifyingly, **123** maintains exceptional potency with an MIC of less than 0.06 µg/mL while the calculated logP decreased to 3.0. Collectively, the results from the last series of 5-aminoquinoline-4-one demonstrate high antibacterial activity can be achieved by introduction of appropriate substituents at C-5 of this scaffold and that the C-5 position is permissive to modification tolerating more polar as well as nonplanar groups.

**Table 8. 5 and 7 Substituted Quinolinones.**

| 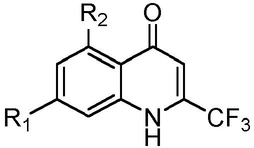 | | | | |
|---|---|---|---|---|
| **Compound** | **R₁** | **R₂** | **MIC µg/mL** | **cLog P** |
| **108** | CF₃ | 3,4-dichloroanilino | <0.06 | 5.2 |
| **109** | CF₃ | 3,4-difluoroanilino | <0.06 | 4.1 |
| **110** | CF₃ | 3-(trifluoromethoxy)anilino | <0.06-0.12 | 4.9 |
| **111** | CF₃ | 5-(trifluoromethyl)pyridin-2-yl-amino | <0.06 | 3.8 |
| **112** | CF₃ | 5-(fluoro)pyridin-2-yl-amino | 0.12 | 3.1 |
| **113** | CF₃ | 5-dimethylaminopyridin-2-yl-amino | 2 | 3.6 |
| **114** | CF₃ | 6-(trifluoromethyl)pyridazine-3-yl- | 1 | 2.9 |
| **115** | CF₃ | (N,N-dimethyl-6-sulfamoyl)pyridin-2- | 2 | 2.1 |
| **116** | CF₃ | 1H-indazol-4-yl-amino | 2 | 3.6 |
| **117** | CF₃ | 4-(trifluoromethyl)cyclohexyl-amino | <0.06 | 3.9 |
| **118** | CF₃ | morpholino | >32 | 2.0 |
| **119** | 3,4-dichloroanilino | CF₃ | 1 | 5.2 |
| **120** | 3,4-difluoroanilino | CF₃ | 4 | 4.1 |
| **121** | 3- | CF₃ | 1 | 4.9 |
| **122** | 5- | CF₃ | 4 | 3.8 |
| **123** | F | 5-(trifluoromethyl)pyridin-2-yl-amino | <0.06 | 3.1 |

Applicants selected a few of the most potent compounds from the 4-aminoquinoline **(22, 31,** not of the invention) and 5-aminoquinolin-4-one **(111, 123)** series for evaluation against a panel of other MRSA strains and representative gram-positive and gram-negative pathogens **(Table 9).** Compounds **111** and **123** show excellent activity (MIC<0.06 µg/mL) towards all six *S. aureus* strains while **31** also displays very good activity with MICs ranging from 0.125-0.25 µg/mL. The compounds maintain activity against *Staphylococcus epidermidis;* however, **31, 111** and **123** all lose considerable potency against *Enterococcus faecalis* and *Enterococcus faecium,* which contribute heavily, along with MRSA, to healthcare-associated infections. The compounds are inactive against the gram-negative bacilli *Escherichia coli, Klebsiella pneumoniae,* and *Enterococcus cloacae* as well as the fungus *Candida albicans* at the highest concentration tested.

**Table 9. Antimicrobial Susceptibility (* = not of the invention).**

| **Species** | **Strain** | **MIC (µg/mL)** | | | |
|---|---|---|---|---|---|
| | | **22*** | **31*** | **111** | **123** |
| | FPR3757 | 0.06 | 0.25 | <0.06 | <0.06 |
| | MW2 | 0.12 5 | 0.12 5 | <0.06 | <0.06 |
| *S. aureus* | COL | - | - | <0.06 | <0.06 |
| | N325 | - | - | <0.06 | <0.06 |
| | NRS71 | - | - | <0.06 | <0.06 |
| | NIH04008 | 0.12 5 | 0.12 5 | <0.06 | <0.06 |
| *S. epidermidis* | NIH04003 | - | - | <0.06 | <0.06 |
| *E. faecalis* | SMC374 | 0.12 *5* | 2 | - | - |
| | DHMC #1 | - | - | 2 | 8 |
| *E. faecium* | ATCC195 79 | 0.12 5 | 0.25-1 | - | - |
| | DHMC #1 | - | - | 2 | 8 |
| *E. coli* | DHMC-1 | >4 | >64 | - | |
| *K. pneumonia* | 7117 | >4 | >64 | - | |
| *E. cloacae* | ND-21 | >4 | >64 | - | |
| *C. albicans* | | >16x MIC | >16x MIC | - | |

While the MIC data confirmed that these compounds were at least bacteriostatic, Applicants wanted to test for bactericidal properties of both the aminoquinoline and aminoquinolone scaffolds. The minimum bactericidal concentration (MBC) of 22, **31, 117** and **123** was evaluated against the MRSA clinical strain *S. aureus* FPR3757. Compounds **22, 31,** and **123** showed potent bactericidal activity with MBCs equal to their MIC values. The kinetics of bacterial killing was assessed in vitro using time-kill assays by incubating compounds at 1× their MIC with an initial inoculum of 10⁵ colony forming units (CFU) of *S. aureus* and removing aliquots at various time points to determine the residual CFU by plating. Compounds **22, 31,** and **123** were bactericidal and reduced the CFU below the limit of detection at four to six hours **(****FIG. 4****).**

### Example 1.5. Mechanism of Action Studies

Classical macromolecular synthesis assays were performed to provide insight on the putative mechanism of action of the most promising quinoline **31** and quinolone **123** candidates. Disruption of bacterial membranes is not only rapid, but interferes with all major metabolic activities in the cell and is thus easily distinguished by macromolecular synthesis assays. Radiolabeled precursors [³H]-L-isoleucine, [³H]-thymidine, [³H]-uridine, and [³H]-glucosamine were added to a culture of *Staphylococcus simulans* (OD₆₀₀ = 0.4) as a surrogate for *S. aureus* in Muellar-Hinton cation (MHC) adjusted medium at 37 °C along with compounds at 0.5×, 1× and 5× MIC. The control antibiotics ciprofloxacin, rifampicin, vancomycin and tetracycline were included as inhibitors of DNA, RNA, cell wall, and protein synthesis, respectively. The cells were quenched at various time points with 10% trichloroacetic acid (TCA), filtered, washed and the amount of precursor incorporation was quantified by scintillation counting. Cells treated with **31** or **123** show a clear concentration-dependent inhibition of DNA, RNA, protein and cell-wall synthesis **(****FIG. 5****).** At 5× the MIC both **31** and **123** completely inhibited all macromolecular processes, a profile that is consistent with disruption of the cellular membrane.

While macromolecular synthesis assays suggested membrane disruption as a likely mechanism, Applicants wanted to provide additional evidence for bacterial membrane damage. Transmission electron microscopy (TEM) and fluorescence microscopy (FM) allowed Applicants to directly observe the effects of the novel 4-aminoquinolines on 5-aminoquinolones on the cell morphology of staphylococci. *S. aureus* (strain USA300) cells treated with **31** and **123** after 10 minutes displayed cross-wall-septum formation with reduced splitting compared to the untreated cells in TEM images **(****FIGS. 6A, 6B,** and **6C****).**

Cells treated with **31** and **123** cells also displayed mesosome-like membrane inclusions, membrane "wrinkling," and bulging of the septum (arrows). These cellular defects were not seen in any of the control cells treated with DMSO. Fluorescent microscopy was performed on a *S. aureus* (strain COL) strain after treatment with either DMSO, **31** or **123** for 30 minutes followed by staining with FM 4-64 (red membrane stain), bodipy-vancomycin (Van-FL, green stain), and Hoechst (blue DNA stain). The red membrane staining showed membrane defects **(****FIG. 7B****)** in the cells treated with **31** and **123** that included large bulges and bulging septum formation. DNA and cell wall staining showed little or no change compared to the DMSO control **(****FIG. 7A****).**

The macromolecular synthesis assay, TEM, and FM experiments were all consistent with membrane disruption. Applicants next sought to determine membrane selectivity of representative 4-aminoquinolines **(31, 76** and **78,** not of the invention) and the most potent 5-aminoquinolin-4-ones **(108-111, 117** and **123)** by a hemolytic assay employing washed sheep erythrocytes. None of the compounds except **76,** displayed any hemolysis at 32 µg/mL compared to the positive control Triton X-100 (100% lysis) **(****FIG. 8****).** The promising 5-aminoquinolin-4-ones tested **(108-111, 117, 123)** displayed greater than 500-fold selectivity for *S. aureus* membranes over erythrocyte membranes. The high membrane selectivity of the 5-aminoquinolin-4-ones in this assay is impressive and is in accordance with the observed therapeutic index (CC₅₀/MIC).

### Example 1.6. Conclusion

The SAR of the quinolone DNAC-2 (MIC = 8 µg/mL) was systematically explored through the synthesis of more than 100 analogues that examined modification to every position of the scaffold. Strongly electron-withdrawing substituents (CF₃ and OCF₃) were optimal at the C-2 and C-7 positions while electron-donating substituents abolished activity. Introduction of a 4-arylamino group at C-4 led to a dramatic increase in potency culminating in 3,4-dichlorophenylamino **18** (not of the invention) with an MIC of 0.06 µg/mL that was offset by a large increase in the calculated logP to 7.7. A great deal of effort was subsequently expended to maintain this outstanding activity while decreasing the lipophilicity and planarity of the molecule. Isosteric replacement of the C-4 group with a more polar 5'-(trifluoromethyl)pyridin-2-yl-amino moiety in **31** (not of the invention) helped to decrease the calculated logP to 6.1 with an attendant 4-fold loss of potency, but non-conservative changes to introduce more polar or non-planar heterocylces were not allowed. Examination of replacements for the lipophilic 2- and 7-trifluoromethyl groups revealed 6,7-difluoro substitution of the quinoline in **78** (not of the invention) was tolerated while reducing the calculated logP to 5.3; however, further attempts to modulate the lipophilicity through introduction of nitrogen atoms into the quinoline scaffold indicated potency and lipophilicity could not be separated. A breakthrough in the SAR was observed by synthesis of a hybrid 5-aminoquinolin-4-one scaffold by combining the quinol-4-one core of DNAC-2 with an N-aryl substituent at C-5, typified by **111** containing an 5'-(trifluoromethyl)pyridin-2-yl-amino group at C-5, whose MIC was less than 0.06 µg/mL with a calculated logP of 3.8. Further refinement led to compound **123** with equipotent activity, but a calculated logP of 3.1.

The 4-aminoquinoline and 5-aminoquinolone scaffolds represented by **31** (not of the invention) and **123** are narrow-spectrum agents with antibacterial activity against strictly gram-positive organisms and no activity against gram-negative bacilli or fungi. Staphylococci including several multidrug-resistant MRSA strains and *S. epidermidis* are most sensitive with MICs of ≤0.06-0.12 µg/mL, but *E. faecalis,* and *E. faecium* are susceptible with MICs ranging from 2-8 µg/mL. The compounds are bactericidal with MBCs equal to the MIC values and were shown to rapidly kill staphylococci reducing the CFUs in vitro by 5-log₁₀ units within the first six hours. The 4-aminoquinoline and 5-aminoquinolone are non-toxic displaying no cytotoxicity at the highest concentration evaluated providing therapeutic indexes of greater than 1000. Preliminary resistance and mechanism of action studies demonstrate these compounds selectively disrupt bacterial membrane. Overall, compound 5-aminoquinolone **123** is the most promising derivative identified from these studies based on its exceptionally potent activity, excellent therapeutic index, selective membrane-disruption and attractive physicochemical properties, which are distinguished from other membrane-active agents that tend to be large amphipathic molecules.

### Example 1.7. High-Throughput Screening (HTS) Small Molecule Screening Assay

Compounds were screened as previously reported. Over 45,000 compounds from a pre-selected small molecule library at the ICCB-Longwood Screening Facility, a part of the New England Regional Centers of Excellence (NERCE), were screened for inhibition of MRSA USA300 growth. OD₆₂₀ was measured in a 384-well format. Treatment of cells with ceftoxin (32 µg/ml) was used as the positive control while cells grown in Mueller-Hinton Broth (MHB) alone were used as the negative control. Per CLSI protocol, the 384-well plates were grown without shaking at 37 °C for 24 hours. Each well was scaled to respective positive and negative control to normalize the percent survival using the following equation: $Percent survival = \frac{\left(OD of the sample-OD of positive control\right)}{\left(OD of negative control-OD of positive control\right)} \times 100$

Compounds yielding <50% survival with USA300 were considered as hits in the primary screen and further validated in the secondary screen where Applicants looked for compounds with at least 80% inhibition in growth.

Without further elaboration, it is believed that one skilled in the art can, using the description herein, utilize the present disclosure to its fullest extent. The embodiments described herein are to be construed as illustrative and not as constraining the remainder of the disclosure in any way whatsoever. Accordingly, the scope of protection is not limited by the description set out above, but is only limited by the claims, including all equivalents of the subject matter of the claims.

## Claims

1. An anti-bacterial compound for use in inhibiting bacterial growth in a subject, wherein the anti-bacterial compound is: 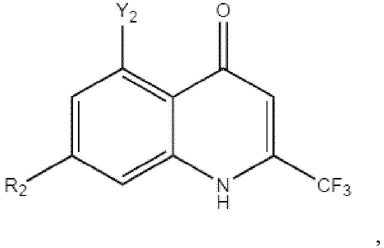 wherein
Y₂ is selected from the group consisting of -S-R₄, -O-R₄, -O-N=CH-R₄, -N(Me)-R₄, - N(C=O)R₄, -NH-R₄, -C(=O)R₄, -C(=O)O-R₄, -NH-R₄-C(=O)OH, -NH-R₄-C(=O)NH₂, -NH-R₄-NO₂, -NH-R₄-CN, -NH-R₄-CF₃, -NH-R₄-F, -NH-R₄-CHF₂, -NH-R₄-CH₂F, -R₄-alky(C₁-C₄), -H, - F, -CF₃, -CHF₂, -CH₂F, alky(C₁-C₄), a heterocyclic, an aromatic, methoxy, thiomethyl, cyano, nitro, chloro, bromo, iodo, cycloalkyl(C₃-C₆), an alkenyl(C₃-C₆) group, a cycloalkenyl(C₃-C₆) group, an alkynyl(C₃-C₆) group, a cycloalkyl(C₃-C₇)oxy, an alkyl(C₁-C₄)oxycarbonyl, alkyl(C₁-C₄), an alkyl(C₁-C₄)oxycarbonyloxymethyl group, a branched alkyl(C₄-C₈)oxycarbonyloxymethyl group, phenyl, an aryl alcohol, a phenylalkyl(C₁-C₄), an aryl halo, a pyridyl, a pyridyl amino, an indolone, a pyridazine, an aryl ketone, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, an aryl thiol, a disulfide, a sulfoxide, a sulfone, a sulfonamide, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, piperazinyl, piperazin-1-yl, morpholino, a thiol, an aryl thiol, a disulfide, a sulfoxide, a sulfone, a sulfonamide, piperazinyl, 3,4-dichloroanilino, 3,4-difluoroanilino, 3-(trifluoromethoxy)aniline, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H-*indazol-4-yl-amino, 4-(trifluoromethyl)cyclohexyl-amino, 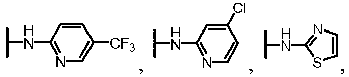 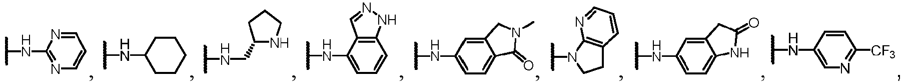 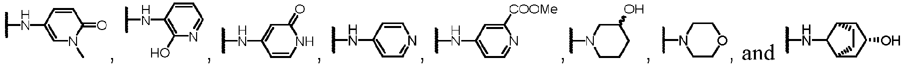
R₂ is selected from the group consisting of H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, alky(C₁-C₄), chloro, ethyl, benzylether, methoxy, thiomethyl, a benzonitrile, a pyridyl, a pyridyl amino, an aniline, an amino, piperazinyl, a pyrrolidine, an indolone, an anilino, a pyridazine, a heterocyclic, an aromatic, an alkyl, a cycloalkyl, an alkenyl, a cycloalkenyl, an alkynyl, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, phenyl, a ketone, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, thiomethyl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, 3,4-dichlorophenyl, 4-trifluoromethylphenyl, 5-(trifluoromethyl)pyridin-2-yl-amino, 3,4-dichloroanilino, 3,4-difluoroanilino, 3-(trifluoromethoxy)aniline, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1H-indazol-4-yl-amino, 4-(trifluoromethyl)cyclohexyl, 4-(trifluoromethyl)cyclohexyl-amino, thiazol-2-yl-amino, and morpholino; and
wherein at least one of Y₂ or R₂ is CF₃.

2. The anti-bacterial compound for use of claim 1, wherein the inhibition of bacterial growth occurs by killing the bacteria, disruption of bacterial cell membranes, rupturing bacterial cell membranes, slowing down bacterial proliferation, or combinations thereof, optionally
wherein the bacteria comprise Gram-positive (Gram⁺) bacteria, further optionally
wherein the bacteria are selected from the group consisting of Gram-positive (Gram⁺) bacteria, antibiotic resistant Gram⁺ bacteria, *Enterococcus faecium, Staphylococcus epidermidis,* methicillin-resistant *Staphylococcus aureus* (MRSA), methicillin-susceptible *Staphylococcus aureus,* or combinations thereof.

3. An anti-bacterial compound comprising: 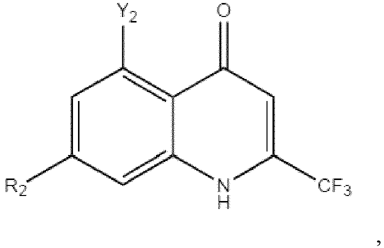 wherein
Y₂ is selected from the group consisting of -S-R₄, -O-R₄, -O-N=CH-R₄, -N(Me)-R₄, - N(C=O)R₄, -NH-R₄, -C(=O)R₄, -C(=O)O-R₄, -NH-R₄-C(=O)OH, -NH-R₄-C(=O)NH₂, -NH-R₄-NO₂, -NH-R₄-CN, -NH-R₄-CF₃, -NH-R₄-F, -NH-R₄-CHF₂, -NH-R₄-CH₂F, -R₄-alky(C₁-C₄), -H, - F, -CF₃, -CHF₂, -CH₂F, alky(C₁-C₄), a heterocyclic, an aromatic, methoxy, thiomethyl, cyano, nitro, chloro, bromo, iodo, cycloalkyl(C₃-C₆), an alkenyl(C₃-C₆) group, a cycloalkenyl(C₃-C₆) group, an alkynyl(C₃-C₆) group, a cycloalkyl(C₃-C₇)oxy, an alkyl(C₁-C₄)oxycarbonyl, alkyl(C₁-C₄), an alkyl(C₁-C₄)oxycarbonyloxymethyl group, a branched alkyl(C₄-C₈)oxycarbonyloxymethyl group, phenyl, an aryl alcohol, a phenylalkyl(C₁-C₄), an aryl halo, a pyridyl, a pyridyl amino, an indolone, a pyridazine, an aryl ketone, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, an aryl thiol, a disulfide, a sulfoxide, a sulfone, a sulfonamide, pyrrol-2-yl, pyrrol-3-yl, pyrrol-2-ylamino, 1-methylpyrrol-2-yl, 1-methylpyrrol-3-yl, 1-methylpyrrol-2-ylamino, morpholino, piperazinyl, piperazin-1-yl, morpholino, a thiol, an aryl thiol, a disulfide, a sulfoxide, a sulfone, a sulfonamide, piperazinyl, 3,4-dichloroanilino, 3,4-difluoroanilino, 3-(trifluoromethoxy)aniline, 5-(trifluoromethyl)pyridin-2-yl-amino, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H-*indazol-4-yl-amino, 4-(trifluoromethyl)cyclohexyl-amino, 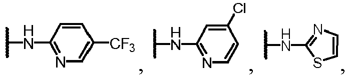 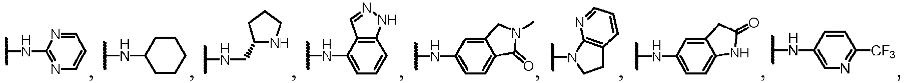 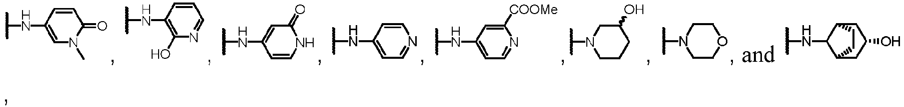
R₂ is selected from the group consisting of H, F, -CHF₂, -CH₂F, -CF₃, -OCF₃, -OMe, - S(O)₂N(Me)₂, -C(=O)OH, tetrazolyl, azido, cyano, nitro, thiomethyl, methoxy, alkyl(C₁-C₄)ester, alky(C₁-C₄), chloro, ethyl, benzylether, methoxy, thiomethyl, a benzonitrile, a pyridyl, a pyridyl amino, an aniline, an amino, piperazinyl, a pyrrolidine, an indolone, an anilino, a pyridazine, a heterocyclic, an aromatic, an alkyl, a cycloalkyl, an alkenyl, a cycloalkenyl, an alkynyl, a cycloalkynyl, an alkyloxy, a cycloalkyloxy, an alkanoyl, an alkyloxycarbonyl, alkyloxycarbonyloxy, an aryl, an aryl alcohol, an aryl alkyl, an aryl halo, a heteroaryl, a heterocycle, phenyl, a ketone, an aryl ketone, an oxime, an aryl oxime, an imine, an aryl imine, an aryl nitrile, an amide, an aryl amide, an aryl nitro, an aryl carbamate, a carbamate, an aldehyde, an aryl aldehyde, a hemiacetal, an aryl hemiacetal, a carboxylic acid, an aryl carboxylic acid, a ester, an aryl ester, an ether, an aryl ether, a thiol, an aryl thiol, a disulfide, a sulfoxide, a sulfone, a sulfonamide, thiomethyl, 3-(trifluoromethoxy)phenyl, 2-isopropylphenyl, 3-acetylphenyl, 3-benzonitrile, 2-chlorophenyl, 3-chlorophenyl, 4-chlorophenyl, 3-fluorophenyl, 3-bromophenyl, 3-iodophenyl, 3,4-difluorophenyl, 3,4-dichloropheyl, 3-(hydroxymethyl)phenyl, 3-thiomethylphenyl, 3-methoxyphenyl, 3-(trifluoromethyl)phenyl, 3,4-(methylenedioxy)phenyl, 3-(morpholino)phenyl, 4-(morpholino)phenyl, 5-(trifluoromethyl)pyridin-2-yl, 3,4-dichlorophenyl, 4-trifluoromethylphenyl, 5-(trifluoromethyl)pyridin-2-yl-amino, 3,4-dichloroanilino, 3,4-difluoroanilino, 3-(trifluoromethoxy)aniline, 5-(fluoro)pyridin-2-yl-amino, 5-dimethylaminopyridin-2-yl-amino, 6-(trifluoromethyl)pyridazine-3-yl-amino, (N,N-dimethyl-6-sulfamoyl)pyridin-2-yl-amino, 1*H*-indazol-4-yl-amino, 4-(trifluoromethyl)cyclohexyl, 4-(trifluoromethyl)cyclohexyl-amino, thiazol-2-yl-amino, and morpholino; and
wherein at least one of Y₂ or R₂ is CF₃.

4. A composition comprising the anti-bacterial compound of claim 3, wherein the composition further comprises one or more active agents, optionally wherein the one or more active agents is oxacillin.

5. The antibacterial compound of claim 3, for use in the treatment or prevention of bacterial infection in the subject.

6. The antibacterial compound of claim 5, for use in the treatment or prevention of bacterial infection in a subject, wherein the compound is for use in intravenous administration, subcutaneous administration, transdermal administration, topical administration, intraarterial administration, intrathecal administration, intracranial administration, intraperitoneal administration, intraspinal administration, intranasal administration, intraocular administration, oral administration, intratumor administration, and combinations thereof.

7. The antibacterial compound of claim 5, for use in the treatment or prevention of bacterial infection in a subject, wherein the anti-bacterial compound is co-administered to the subject with one or more active agents, optionally wherein the one or more active agents is oxacillin.

8. The anti-bacterial compound for use of claim 1, wherein the bacteria comprise Gram-positive (Gram⁺) bacteria, optionally
wherein the bacteria are selected from the group consisting of Gram-positive (Gram⁺) bacteria, antibiotic resistant Gram⁺ bacteria, *Enterococcus faecium, Staphylococcus epidermidis,* methicillin-resistant *Staphylococcus aureus* (MRSA), methicillin-susceptible *Staphylococcus aureus,* or combinations thereof.

## Patentansprüche

1. Eine antibakterielle Verbindung zur Verwendung bei der Hemmung des Bakterienwachstums bei einem Subjekt, wobei die antibakterielle Verbindung Folgendes ist: 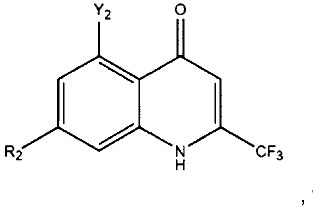 wobei
Y₂ ausgewählt ist aus der Gruppe bestehend aus -S-R₄, -O-R₄, -O-N=CH-R₄, -N(Me)-R₄, -N(C=O)R₄, -NH-R₄, -C(=O)R₄, -C(=O)O-R₄, -NH-R₄-C(=O)OH, -NH-R₄-C(=O)NH₂, - NH-R₄-NO₂, -NH-R₄-CN, -NH-R₄-CF₃, -NH-R₄-F, -NH-R₄-CHF₂, -NH-R₄-CH₂F, -R₄-Alky(C₁-C₄), -H, -F, -CF₃, -CHF₂, -CH₂F, Alky(C₁-C₄), einer heterocyclischen Gruppe, einem Aromaten, Methoxy, Thiomethyl, Cyano, Nitro, Chlor, Brom, Iod, Cycloalkyl(C₃-C₆), einer Alkenyl(C₃-C₆)-Gruppe, einer Cycloalkenyl(C₃-C₆)-Gruppe, einer Alkinyl(C₃-C₆)-Gruppe, einem Cycloalkyl(C₃-C₇)oxy, einem Alkyl(C₁-C₄)oxycarbonyl, Alkyl(C₁-C₄), einer Alkyl(C₁-C₄)oxycarbonyloxymethyl-Gruppe, einer verzweigten Alkyl(C₄-C₈)oxycarbonyloxymethyl-Gruppe, Phenyl, einem Arylalkohol, einem Phenylalkyl(C₁-C₄), einem Arylhalogen, einem Pyridyl, einem Pyridylamino, einem Indolon, einem Pyridazin, einem Arylketon, einem Aryloxim, einem Imin, einem Arylimin, einem Arylnitril, einem Amid, einem Arylamid, einem Arylnitro, einem Arylcarbamat, einem Carbamat, einem Aldehyd, einem Arylaldehyd, einem Hemiacetal, einem Arylhemiacetal, einem Arylthiol, einem Disulfid, einem Sulfoxid, einem Sulfon, einem Sulfonamid, Pyrrol-2-yl, Pyrrol-3-yl, Pyrrol-2-ylamino, 1-Methylpyrrol-2-yl, 1-Methylpyrrol-3-yl, 1-Methylpyrrol-2-ylamino, Morpholino, Piperazinyl, Piperazin-1-yl, Morpholino, einem Thiol, einem Arylthiol, einem Disulfid, einem Sulfoxid, einem Sulfon, einem Sulfonamid, Piperazinyl, 3,4-Dichloranilino, 3,4-Difluoranilino, 3-(Trifluormethoxy)anilin, 5-(Trifluormethyl)pyridin-2-yl-amino, 5-(Fluor)pyridin-2-yl-amino, 5-Dimethylaminopyridin-2-yl-amino, 6-(Trifluormethyl)pyridazin-3-yl-amino, (N,N-Dimethyl-6-sulfamoyl)pyridin-2-yl-amino, 1*H*-Indazol-4-yl-amino, 4-(Trifluormethyl)cyclohexyl-amino, 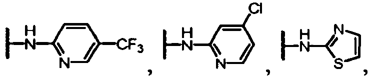 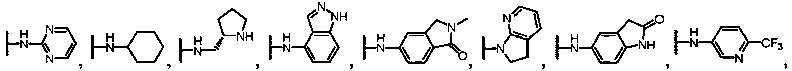 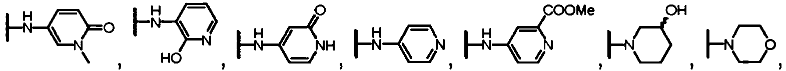 und 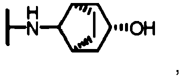
R₂ ausgewählt ist aus der Gruppe bestehend aus H, F, -CHF₂, -CH₂F, -CF₃, -OCF₃, - OMe, -S(O)₂N(Me)₂, -C(=O)OH, Tetrazolyl, Azido, Cyano, Nitro, Thiomethyl, Methoxy, Alkyl(C₁-C₄)ester, Alky(C₁-C₄), Chlor, Ethyl, Benzylether, Methoxy, Thiomethyl, einem Benzonitril, einem Pyridyl, einem Pyridylamino, einem Anilin, einem Amino, Piperazinyl, einem Pyrrolidin, einem Indolon, einem Anilino, einem Pyridazin, einer heterocyclischen Gruppe, einem Aromaten, einem Alkyl, einem Cycloalkyl, einem Alkenyl, einem Cycloalkenyl, einem Alkinyl, einem Cycloalkinyl, einem Alkyloxy, einem Cycloalkyloxy, einem Alkanoyl, einem Alkyloxycarbonyl, Alkyloxycarbonyloxy, einem Aryl, einem Arylalkohol, einem Arylalkyl, einem Arylhalogen, einem Heteroaryl, einem Heterocyclus, Phenyl, einem Keton, einem Arylketon, einem Oxim, einem Aryloxim, einem Imin, einem Arylimin, einem Arylnitril, einem Amid, einem Arylamid, einem Arylnitro, einem Arylcarbamat, einem Carbamat, einem Aldehyd, einem Arylaldehyd, einem Hemiacetal, einem Arylhemiacetal, einer Carbonsäure, einer Arylcarbonsäure, einem Ester, einem Arylester, einem Ether, einem Arylether, einem Thiol, einem Arylthiol, einem Disulfid, einem Sulfoxid, einem Sulfon, einem Sulfonamid, Thiomethyl, 3-(Trifluormethoxy)phenyl, 2-Isopropylphenyl, 3-Acetylphenyl, 3-Benzonitril, 2-Chlorphenyl, 3-Chlorphenyl, 4-Chlorphenyl, 3-Fluorphenyl, 3-Bromphenyl, 3-Iodphenyl, 3,4-Difluorphenyl, 3,4-Dichlorphenyl, 3-(Hydroxymethyl)phenyl, 3-Thiomethylphenyl, 3-Methoxyphenyl, 3-(Trifluormethyl)phenyl, 3,4-(Methylendioxy)phenyl, 3-(Morpholino)phenyl, 4-(Morpholino)phenyl, 5-(Trifluormethyl)pyridin-2-yl, 3,4-Dichlorphenyl, 4-Trifluormethylphenyl, 5-(Trifluormethyl)pyridin-2-yl-amino, 3,4-Dichloranilino, 3,4-Difluoranilino, 3-(Trifluormethoxy)anilin, 5-(Fluor)pyridin-2-yl-amino, 5-Dimethylaminopyridin-2-yl-amino, 6-(Trifluormethyl)pyridazin-3-yl-amino, (N,N-Dimethyl-6-sulfamoyl)pyridin-2-yl-amino, 1*H*-Indazol-4-yl-amino, 4-(Trifluormethyl)cyclohexyl, 4-(Trifluormethyl)cyclohexyl-amino, Thiazol-2-yl-amino und Morpholino; und
wobei mindestens eines von Y₂ oder R₂ CF₃ ist.

2. Antibakterielle Verbindung zur Verwendung gemäß Anspruch 1, wobei die Hemmung des Bakterienwachstums durch Abtöten der Bakterien, Störung von Bakterienzellmembranen, Aufbrechen von Bakterienzellmembranen, Verlangsamen von bakterieller Proliferation oder Kombinationen davon erfolgt,
wobei die Bakterien gegebenenfalls grampositive (Gram⁺) Bakterien beinhalten,
wobei die Bakterien ferner gegebenenfalls ausgewählt sind aus der Gruppe bestehend aus grampositiven (Gram⁺) Bakterien, antibiotikaresistenten Gram⁺-Bakterien, *Enterococcus faecium*, *Staphylococcus epidermidis*, methicillinresistentem *Staphylococcus aureus* (MRSA), methicillinempfindlichem *Staphylococcus aureus* oder Kombinationen davon.

3. Antibakterielle Verbindung, beinhaltend: 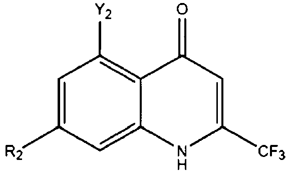 wobei
Y₂ ausgewählt ist aus der Gruppe bestehend aus -S-R₄, -O-R₄, -O-N=CH-R₄, -N(Me)-R₄, -N(C=O)R₄, -NH-R₄, -C(=O)R₄, -C(=O)O-R₄, -NH-R₄-C(=O)OH, -NH-R₄-C(=O)NH₂, - NH-R₄-NO₂, -NH-R₄-CN, -NH-R₄-CF₃, -NH-R₄-F, -NH-R₄-CHF₂, -NH-R₄-CH₂F, -R₄-Alky(C₁-C₄), -H, -F, -CF₃, -CHF₂, -CH₂F, Alky(C₁-C₄), einer heterocyclischen Gruppe, einem Aromaten, Methoxy, Thiomethyl, Cyano, Nitro, Chlor, Brom, Iod, Cycloalkyl(C₃-C₆), einer Alkenyl(C₃-C₆)-Gruppe, einer Cycloalkenyl(C₃-C₆)-Gruppe, einer Alkinyl(C₃-C₆)-Gruppe, einem Cycloalkyl(C₃-C₇)oxy, einem Alkyl(C₁-C₄)oxycarbonyl, Alkyl(C₁-C₄), einer Alkyl(C₁-C₄)oxycarbonyloxymethyl-Gruppe, einer verzweigten Alkyl(C₄-C₈)oxycarbonyloxymethyl-Gruppe, Phenyl, einem Arylalkohol, einem Phenylalkyl(C₁-C₄), einem Arylhalogen, einem Pyridyl, einem Pyridylamino, einem Indolon, einem Pyridazin, einem Arylketon, einem Aryloxim, einem Imin, einem Arylimin, einem Arylnitril, einem Amid, einem Arylamid, einem Arylnitro, einem Arylcarbamat, einem Carbamat, einem Aldehyd, einem Arylaldehyd, einem Hemiacetal, einem Arylhemiacetal, einem Arylthiol, einem Disulfid, einem Sulfoxid, einem Sulfon, einem Sulfonamid, Pyrrol-2-yl, Pyrrol-3-yl, Pyrrol-2-ylamino, 1-Methylpyrrol-2-yl, 1-Methylpyrrol-3-yl, 1-Methylpyrrol-2-ylamino, Morpholino, Piperazinyl, Piperazin-1-yl, Morpholino, einem Thiol, einem Arylthiol, einem Disulfid, einem Sulfoxid, einem Sulfon, einem Sulfonamid, Piperazinyl, 3,4-Dichloranilino, 3,4-Difluoranilino, 3-(Trifluormethoxy)anilin, 5-(Trifluormethyl)pyridin-2-yl-amino, 5-(Fluor)pyridin-2-yl-amino, 5-Dimethylaminopyridin-2-yl-amino, 6-(Trifluormethyl)pyridazin-3-yl-amino, (N,N-Dimethyl-6-sulfamoyl)pyridin-2-yl-amino, 1*H*-Indazol-4-yl-amino, 4-(Trifluormethyl)cyclohexyl-amino, 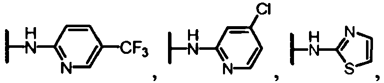 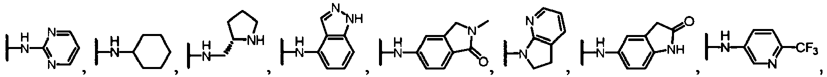 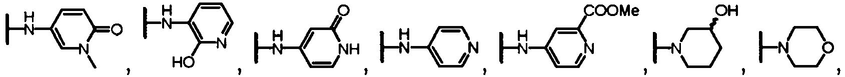 und 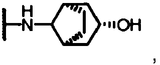
R₂ ausgewählt ist aus der Gruppe bestehend aus H, F, -CHF₂, -CH₂F, -CF₃, -OCF₃, - OMe, -S(O)₂N(Me)₂, -C(=O)OH, Tetrazolyl, Azido, Cyano, Nitro, Thiomethyl, Methoxy, Alkyl(C₁-C₄)ester, Alky(C₁-C₄), Chlor, Ethyl, Benzylether, Methoxy, Thiomethyl, einem Benzonitril, einem Pyridyl, einem Pyridylamino, einem Anilin, einem Amino, Piperazinyl, einem Pyrrolidin, einem Indolon, einem Anilino, einem Pyridazin, einer heterocyclischen Gruppe, einem Aromaten, einem Alkyl, einem Cycloalkyl, einem Alkenyl, einem Cycloalkenyl, einem Alkinyl, einem Cycloalkinyl, einem Alkyloxy, einem Cycloalkyloxy, einem Alkanoyl, einem Alkyloxycarbonyl, Alkyloxycarbonyloxy, einem Aryl, einem Arylalkohol, einem Arylalkyl, einem Arylhalogen, einem Heteroaryl, einem Heterocyclus, Phenyl, einem Keton, einem Arylketon, einem Oxim, einem Aryloxim, einem Imin, einem Arylimin, einem Arylnitril, einem Amid, einem Arylamid, einem Arylnitro, einem Arylcarbamat, einem Carbamat, einem Aldehyd, einem Arylaldehyd, einem Hemiacetal, einem Arylhemiacetal, einer Carbonsäure, einer Arylcarbonsäure, einem Ester, einem Arylester, einem Ether, einem Arylether, einem Thiol, einem Arylthiol, einem Disulfid, einem Sulfoxid, einem Sulfon, einem Sulfonamid, Thiomethyl, 3-(Trifluormethoxy)phenyl, 2-Isopropylphenyl, 3-Acetylphenyl, 3-Benzonitril, 2-Chlorphenyl, 3-Chlorphenyl, 4-Chlorphenyl, 3-Fluorphenyl, 3-Bromphenyl, 3-Iodphenyl, 3,4-Difluorphenyl, 3,4-Dichlorphenyl, 3-(Hydroxymethyl)phenyl, 3-Thiomethylphenyl, 3-Methoxyphenyl, 3-(Trifluormethyl)phenyl, 3,4-(Methylendioxy)phenyl, 3-(Morpholino)phenyl, 4-(Morpholino)phenyl, 5-(Trifluormethyl)pyridin-2-yl, 3,4-Dichlorphenyl, 4-Trifluormethylphenyl, 5-(Trifluormethyl)pyridin-2-yl-amino, 3,4-Dichloranilino, 3,4-Difluoranilino, 3-(Trifluormethoxy)anilin, 5-(Fluor)pyridin-2-yl-amino, 5-Dimethylaminopyridin-2-yl-amino, 6-(Trifluormethyl)pyridazin-3-yl-amino, (N,N-Dimethyl-6-sulfamoyl)pyridin-2-yl-amino, 1*H*-Indazol-4-yl-amino, 4-(Trifluormethyl)cyclohexyl, 4-(Trifluormethyl)cyclohexyl-amino, Thiazol-2-yl-amino und Morpholino; und
wobei mindestens eines von Y₂ oder R₂ CF₃ ist.

4. Eine Zusammensetzung, beinhaltend die antibakterielle Verbindung gemäß Anspruch 3, wobei die Zusammensetzung ferner einen oder mehrere Wirkstoffe beinhaltet, wobei es sich bei dem einen oder den mehreren Wirkstoffen gegebenenfalls um Oxacillin handelt.

5. Antibakterielle Verbindung gemäß Anspruch 3 zur Verwendung bei der Behandlung oder Prävention einer bakteriellen Infektion bei dem Subjekt.

6. Antibakterielle Verbindung gemäß Anspruch 5 zur Verwendung bei der Behandlung oder Prävention einer bakteriellen Infektion bei einem Subjekt, wobei die Verbindung zur Verwendung bei intravenöser Verabreichung, subkutaner Verabreichung, transdermaler Verabreichung, topischer Verabreichung, intraarterieller Verabreichung, intrathekaler Verabreichung, intrakranialer Verabreichung, intraperitonealer Verabreichung, intraspinaler Verabreichung, intranasaler Verabreichung, intraokularer Verabreichung, oraler Verabreichung, intratumoraler Verabreichung und Kombinationen davon ist.

7. Antibakterielle Verbindung gemäß Anspruch 5 zur Verwendung bei der Behandlung oder Prävention einer bakteriellen Infektion bei einem Subjekt, wobei die antibakterielle Verbindung dem Subjekt zusammen mit einem oder mehreren Wirkstoffen verabreicht wird, wobei es sich bei dem einen oder den mehreren Wirkstoffen gegebenenfalls um Oxacillin handelt.

8. Antibakterielle Verbindung zur Verwendung gemäß Anspruch 1, wobei die Bakterien grampositive (Gram⁺) Bakterien beinhalten,
wobei die Bakterien gegebenenfalls ausgewählt sind aus der Gruppe bestehend aus grampositiven (Gram⁺) Bakterien, antibiotikaresistenten Gram⁺-Bakterien, *Enterococcus faecium*, *Staphylococcus epidermidis*, methicillinresistentem *Staphylococcus aureus* (MRSA), methicillinempfindlichem *Staphylococcus aureus* oder Kombinationen davon.

## Revendications

1. Un composé antibactérien pour son utilisation dans l'inhibition de la croissance bactérienne chez un sujet, où le composé antibactérien est : 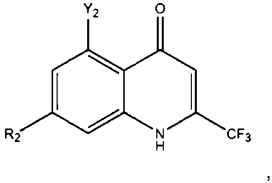 où
Y₂ est sélectionné dans le groupe constitué de -S-R₄, -O-R₄, -O-N=CH-R₄, -N(Me)-R₄, -N(C=O)R₄, -NH-R₄, -C(=O)R₄, -C(=O)O-R₄, -NH-R₄-C(=O)OH, -NH-R₄-C(=O)NH₂, -NH-R₄-NO₂, -NH-R₄-CN, -NH-R₄-CF₃, -NH-R₄-F, -NH-R₄-CHF₂, -NH-R₄-CH₂F, -R₄-alky(C₁-C₄), -H, -F, -CF₃, -CHF₂, -CH₂F, alky(C₁-C₄), un hétérocyclique, un aromatique, méthoxy, thiométhyle, cyano, nitro, chloro, bromo, iodo, cycloalkyl(C₃-C₆), un groupe alcényl(C₃-C₆), un groupe cycloalcényl(C₃-C₆), un groupe alcynyl(C₃-C₆), un cycloalkyl(C₃-C₇)oxy, un alkyl(C₁-C₄)oxycarbonyle, alkyl(C₁-C₄), un groupe alkyl(C₁-C₄)oxycarbonyloxyméthyle, un groupe alkyl(C₄-C₈)oxycarbonyloxyméthyle ramifié, phényle, un arylalcool, un phénylalkyl(C₁-C₄), un arylhalo, un pyridyle, un pyridylamino, un indolone, une pyridazine, une arylcétone, une aryloxime, une imine, une arylimine, un arylnitrile, un amide, un arylamide, un arylnitro, un arylcarbamate, un carbamate, un aldéhyde, un arylaldéhyde, un hémiacétal, un arylhémiacétal, un arylthiol, un disulfure, un sulfoxyde, un sulfone, un sulfonamide, pyrrol-2-yle, pyrrol-3-yle, pyrrol-2-ylamino, 1-méthylpyrrol-2-yle, 1-méthylpyrrol-3-yle, 1-méthylpyrrol-2-ylamino, morpholino, pipérazinyle, pipérazin-1-yle, morpholino, un thiol, un arylthiol, un disulfure, un sulfoxyde, un sulfone, un sulfonamide, pipérazinyle, 3,4-dichloroanilino, 3,4-difluoroanilino, 3-(trifluorométhoxy)aniline, 5-(trifluorométhyl)pyridin-2-yl-amino, 5-(fluoro)pyridin-2-yl-amino, 5-diméthylaminopyridin-2-yl-amino, 6-(trifluorométhyl)pyridazin-3-yl-amino, (N,N-diméthyl-6-sulfamoyl)pyridin-2-yl-amino, 1*H-*indazol-4-yl-amino, 4-(trifluorométhyl)cyclohexyl-amino, 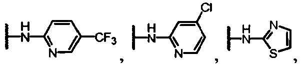 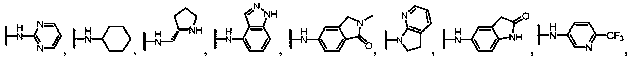 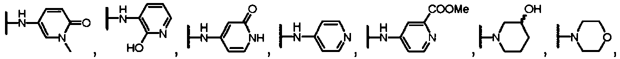 et 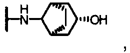
R₂ est sélectionné dans le groupe constitué de H, F, -CHF₂, -CH₂F, -CF₃, -OCF₃, -OMe, -S(O)₂N(Me)₂, -C(=O)OH, tétrazolyle, azido, cyano, nitro, thiométhyle, méthoxy, alkyl(C₁-C₄)ester, alky(C₁-C₄), chloro, éthyle, benzyléther, méthoxy, thiométhyle, un benzonitrile, un pyridyle, un pyridylamino, une aniline, un amino, pipérazinyle, une pyrrolidine, une indolone, un anilino, une pyridazine, un hétérocyclique, un aromatique, un alkyle, un cycloalkyle, un alcényle, un cycloalcényle, un alcynyle, un cycloalcynyle, un alkyloxy, un cycloalkyloxy, un alcanoyle, un alkyloxycarbonyle, alkyloxycarbonyloxy, un aryle, un arylalcool, un arylalkyle, un arylhalo, un hétéroaryle, un hétérocycle, phényle, une cétone, une arylcétone, une oxime, une aryloxime, une imine, une arylimine, un arylnitrile, un amide, un arylamide, un arylnitro, un arylcarbamate, un carbamate, un aldéhyde, un arylaldéhyde, un hémiacétal, un arylhémiacétal, un acide carboxylique, un acide arylcarboxylique, un ester, un arylester, un éther, un aryléther, un thiol, un arylthiol, un disulfure, un sulfoxyde, un sulfone, un sulfonamide, thiométhyle, 3-(trifluorométhoxy)phényle, 2-isopropylphényle, 3-acétylphényle, 3-benzonitrile, 2-chlorophényle, 3-chlorophényle, 4-chlorophényle, 3-fluorophényle, 3-bromophényle, 3-iodophényle, 3,4-difluorophényle, 3,4-dichlorophényle, 3-(hydroxyméthyl)phényle, 3-thiométhylphényle, 3-méthoxyphényle, 3-(trifluorométhyl)phényle, 3,4-(méthylènedioxy)phényle, 3-(morpholino)phényle, 4-(morpholino)phényle, 5-(trifluorométhyl)pyridin-2-yle, 3,4-dichlorophényle, 4-trifluorométhylphényle, 5-(trifluorométhyl)pyridin-2-yl-amino, 3,4-dichloroanilino, 3,4-difluoroanilino, 3-(trifluorométhoxy)aniline, 5-(fluoro)pyridin-2-yl-amino, 5-diméthylaminopyridin-2-yl-amino, 6-(trifluorométhyl)pyridazin-3-yl-amino, (N,N-diméthyl-6-sulfamoyl)pyridin-2-yl-amino, 1*H*-indazol-4-yl-amino, 4-(trifluorométhyl)cyclohexyle, 4-(trifluorométhyl)cyclohexyl-amino, thiazol-2-yl-amino, et morpholino; et
où au moins l'un de Y₂ ou R₂ est CF₃.

2. Le composé antibactérien pour son utilisation de la revendication 1, où l'inhibition de la croissance bactérienne se produit en tuant les bactéries, en perturbant des membranes cellulaires bactériennes, en rompant des membranes cellulaires bactériennes, en ralentissant la prolifération bactérienne, ou par des combinaisons de ce qui précède, facultativement
où les bactéries comprennent des bactéries à Gram positif (Gram⁺), en outre facultativement
où les bactéries sont sélectionnées dans le groupe constitué de bactéries à Gram positif (Gram⁺), de bactéries à Gram⁺ résistantes aux antibiotiques, d'*Enterococcus faecium*, de *Staphylococcus epidermidis*, de *Staphylococcus aureus* résistant à la méticilline (MRSA), de *Staphylococcus aureus* sensible à la méticilline, ou de combinaisons de ceux-ci.

3. Un composé antibactérien comprenant : 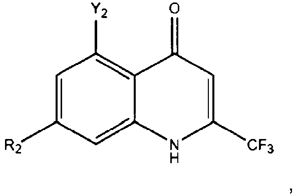 où
Y₂ est sélectionné dans le groupe constitué de -S-R₄, -O-R₄, -O-N=CH-R₄, -N(Me)-R₄, -N(C=O)R₄, -NH-R₄, -C(=O)R₄, -C(=O)O-R₄, -NH-R₄-C(=O)OH, -NH-R₄-C(=O)NH₂, -NH-R₄-NO₂, -NH-R₄-CN, -NH-R₄-CF₃, -NH-R₄-F, -NH-R₄-CHF₂, -NH-R₄-CH₂F, -R₄-alky(C₁-C₄), -H, -F, -CF₃, -CHF₂, -CH₂F, alky(C₁-C₄), un hétérocyclique, un aromatique, méthoxy, thiométhyle, cyano, nitro, chloro, bromo, iodo, cycloalkyl(C₃-C₆), un groupe alcényl(C₃-C₆), un groupe cycloalcényl(C₃-C₆), un groupe alcynyl(C₃-C₆), un cycloalkyl(C₃-C₇)oxy, un alkyl(C₁-C₄)oxycarbonyle, alkyl(C₁-C₄), un groupe alkyl(C₁-C₄)oxycarbonyloxyméthyle, un groupe alkyl(C₄-C₈)oxycarbonyloxyméthyle ramifié, phényle, un arylalcool, un phénylalkyl(C₁-C₄), un arylhalo, un pyridyle, un pyridylamino, un indolone, une pyridazine, une arylcétone, une aryloxime, une imine, une arylimine, un arylnitrile, un amide, un arylamide, un arylnitro, un arylcarbamate, un carbamate, un aldéhyde, un arylaldéhyde, un hémiacétal, un arylhémiacétal, un arylthiol, un disulfure, un sulfoxyde, un sulfone, un sulfonamide, pyrrol-2-yle, pyrrol-3-yle, pyrrol-2-ylamino, 1-méthylpyrrol-2-yle, 1-méthylpyrrol-3-yle, 1-méthylpyrrol-2-ylamino, morpholino, pipérazinyle, pipérazin-1-yle, morpholino, un thiol, un arylthiol, un disulfure, un sulfoxyde, un sulfone, un sulfonamide, pipérazinyle, 3,4-dichloroanilino, 3,4-difluoroanilino, 3-(trifluorométhoxy)aniline, 5-(trifluorométhyl)pyridin-2-yl-amino, 5-(fluoro)pyridin-2-yl-amino, 5-diméthylaminopyridin-2-yl-amino, 6-(trifluorométhyl)pyridazin-3-yl-amino, (N,N-diméthyl-6-sulfamoyl)pyridin-2-yl-amino, 1*H-*indazol-4-yl-amino, 4-(trifluorométhyl)cyclohexyl-amino, 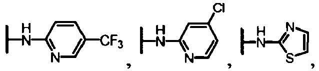 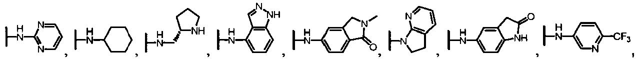 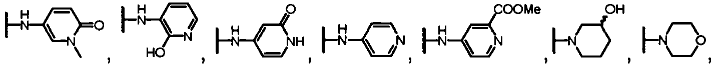 et 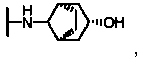
R₂ est sélectionné dans le groupe constitué de H, F, -CHF₂, -CH₂F, -CF₃, -OCF₃, -OMe, -S(O)₂N(Me)₂, -C(=O)OH, tétrazolyle, azido, cyano, nitro, thiométhyle, méthoxy, alkyl(C₁-C₄)ester, alky(C₁-C₄), chloro, éthyle, benzyléther, méthoxy, thiométhyle, un benzonitrile, un pyridyle, un pyridylamino, une aniline, un amino, pipérazinyle, une pyrrolidine, une indolone, un anilino, une pyridazine, un hétérocyclique, un aromatique, un alkyle, un cycloalkyle, un alcényle, un cycloalcényle, un alcynyle, un cycloalcynyle, un alkyloxy, un cycloalkyloxy, un alcanoyle, un alkyloxycarbonyle, alkyloxycarbonyloxy, un aryle, un arylalcool, un arylalkyle, un arylhalo, un hétéroaryle, un hétérocycle, phényle, une cétone, une arylcétone, une oxime, une aryloxime, une imine, une arylimine, un arylnitrile, un amide, un arylamide, un arylnitro, un arylcarbamate, un carbamate, un aldéhyde, un arylaldéhyde, un hémiacétal, un arylhémiacétal, un acide carboxylique, un acide arylcarboxylique, un ester, un arylester, un éther, un aryléther, un thiol, un arylthiol, un disulfure, un sulfoxyde, un sulfone, un sulfonamide, thiométhyle, 3-(trifluorométhoxy)phényle, 2-isopropylphényle, 3-acétylphényle, 3-benzonitrile, 2-chlorophényle, 3-chlorophényle, 4-chlorophényle, 3-fluorophényle, 3-bromophényle, 3-iodophényle, 3,4-difluorophényle, 3,4-dichlorophényle, 3-(hydroxyméthyl)phényle, 3-thiométhylphényle, 3-méthoxyphényle, 3-(trifluorométhyl)phényle, 3,4-(méthylènedioxy)phényle, 3-(morpholino)phényle, 4-(morpholino)phényle, 5-(trifluorométhyl)pyridin-2-yle, 3,4-dichlorophényle, 4-trifluorométhylphényle, 5-(trifluorométhyl)pyridin-2-yl-amino, 3,4-dichloroanilino, 3,4-difluoroanilino, 3-(trifluorométhoxy)aniline, 5-(fluoro)pyridin-2-yl-amino, 5-diméthylaminopyridin-2-yl-amino, 6-(trifluorométhyl)pyridazin-3-yl-amino, (N,N-diméthyl-6-sulfamoyl)pyridin-2-yl-amino, 1*H*-indazol-4-yl-amino, 4-(trifluorométhyl)cyclohexyle, 4-(trifluorométhyl)cyclohexyl-amino, thiazol-2-yl-amino, et morpholino; et
où au moins l'un de Y₂ ou R₂ est CF₃.

4. Une composition comprenant le composé antibactérien de la revendication 3, où la composition comprend en outre un ou plusieurs agents actifs, facultativement où les un ou plusieurs agents actifs sont l'oxacilline.

5. Le composé antibactérien de la revendication 3, pour son utilisation dans le traitement ou la prévention d'une infection bactérienne chez le sujet.

6. Le composé antibactérien de la revendication 5, pour son utilisation dans le traitement ou la prévention d'une infection bactérienne chez un sujet, où le composé est destiné à une utilisation dans l'administration intraveineuse, l'administration sous-cutanée, l'administration transdermique, l'administration topique, l'administration intra-artérielle, l'administration intrathécale, l'administration intracrânienne, l'administration intrapéritonéale, l'administration intraspinale, l'administration intranasale, l'administration intraoculaire, l'administration orale, l'administration intratumorale, et des combinaisons de celles-ci.

7. Le composé antibactérien de la revendication 5, pour son utilisation dans le traitement ou la prévention d'une infection bactérienne chez un sujet, où le composé antibactérien est co-administré au sujet avec un ou plusieurs agents actifs, facultativement où les un ou plusieurs agents actifs sont l'oxacilline.

8. Le composé antibactérien pour son utilisation de la revendication 1, où les bactéries comprennent des bactéries à Gram positif (Gram⁺), facultativement
où les bactéries sont sélectionnées dans le groupe constitué de bactéries à Gram positif (Gram⁺), de bactéries à Gram⁺ résistantes aux antibiotiques, d'*Enterococcus faecium*, de *Staphylococcus epidermidis*, de *Staphylococcus aureus* résistant à la méticilline (MRSA), de *Staphylococcus aureus* sensible à la méticilline, ou de combinaisons de ceux-ci.
